(19) Europäisches Patentamt
European Patent Office
Office européen des brevets

(11) Numéro de publication: **0 419 542 B1**

(12) **FASCICULE DE BREVET EUROPEEN**

(45) Date de publication de fascicule du brevet: **29.09.93**

(51) Int. Cl.5: **C07D 491/16**, //(C07D491/16, 311:00,221:00)

(21) Numéro de dépôt: **89907177.3**

(22) Date de dépôt: **02.06.89**

(86) Numéro de dépôt internationale :
**PCT/FR89/00277**

(87) Numéro de publication internationale :
**WO 89/12052 (14.12.89 89/29)**

(54) **NOUVEAUX DERIVES DE TETRAHYDRO-2,3,6,7,1H,5H,11H-(1)BENZOPYRANO(6,7,8,ij)OUINOLIZINONE-11 UTILISABLES COMME MAROUEURS DE COMPOSES ORGANIOUES EN VUE DE LA DETECTION DE CES COMPOSES PAR CHIMILUMINESCENCE OU FLUORESCENCE.**

(30) Priorité: **02.06.88 FR 8807355**

(43) Date de publication de la demande:
**03.04.91 Bulletin 91/14**

(45) Mention de la délivrance du brevet:
**29.09.93 Bulletin 93/39**

(84) Etats contractants désignés:
**AT BE CH DE FR GB IT LI LU NL SE**

(56) Documents cités:

**Optics Communications, vol. 15, No. 3, November/December 1975 (Amsterdam, NL), K.H. Drexhage et al.: "Water-soluble coumarin dyes for flashlamp-pumped dye lasers", pages 399-403, see page 403, fig. 7**

(73) Titulaire: **LABORATOIRES EUROBIO**
**20, boulevard St. Germain**
**F-75005 Paris(FR)**

(72) Inventeur: **MAHUZIER, Georges**
**33, bld Lefèbvre**
**F-75015 Paris(FR)**
Inventeur: **CHALOM, Joseph**
**1, place Jacques-Blumenthal**
**F-93800 Epinay-sur-Seine(FR)**
Inventeur: **FARINOTTI, Robert**
**Faculté de Pharmacie Rue J.-B.-Clément**
**F-92290 Châtenay-Malabry(FR)**
Inventeur: **TOD, Michel**
**4, rue Fontaine**
**F-75009 Paris(FR)**

(74) Mandataire: **Des Termes, Monique et al**
**Société Brevatome 25, rue de Ponthieu**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

**Journal of Chromatography, vol. 225, 1981, Elsevier Scientific Publishing Co. (Amsterdam, NL), H. Cisse et al.: "Dosage de l'acide dipropylacétique dans le plasma par chromatographie en phase liquide et détection spectrofluorimétrique", pages 509-515, see the whole document**

## Description

La présente invention concerne de nouveaux marqueurs, utilisables en particulier pour le marquage de composés organiques comportant des groupements -NH-, $-NH_2$, -CHO, -COOH ou -CO-, en vue de la détection de ces composés par chimiluminescence ou fluorescence dans des procédés de dosages tels que les dosages par chromatographie liquide.

On sait que le dosage par chromatographie liquide d'amines, d'aldéhydes, de cétones ou d'acides dépourvus de propriétés spectrales nécessite, pour atteindre une grande sensibilité, de transformer ces composés à l'aide de réactifs marqueurs pour leur conférer des propriétés d'absorption, de fluorescence ou de chimiluminescence.

L'utilisation de marqueurs de ce type pour la détection de dérivés carboxyliques ou d'amines est décrite en particulier par H. Cisse, R. Farinotti, S. Kirkiacharian et A. Dauphin dans Journal of Chromatography, 225(1981), p. 509-515 ; par M. Tsitini Tsamis, A.M. Mange, R. Farinotti et G. Mahuzier dans Journal of Chromatography 277(1983), p. 61-69 ; par N. Kubab, R. Farinotti et G. Mahuzier dans Analusis, 1986, v.14, n°3, p. 125-130 ; et par D. Amir et E. Haas dans Int. J. Peptide Protein Res. 26(1986), p. 7-17.

Dans les procédés de dosage par chromatographie liquide,on recherche en particulier des marqueurs chimiluminescents car l'analyse par chimiluminescence est en plein développement et permet d'améliorer la sensibilité du dosage. En effet, les méthodes par chimiluminescence permettent d'atteindre une limite de détection de l'ordre de la femtomole, alors que, dans les méthodes par spectrofluorimétrie, la limite de détection atteinte est couramment de l'ordre de quelques centaines de femtomoles.

Une méthode de détection par chimiluminescence en chromatographie en phase liquide est décrite en particulier par M. Tod, R. Farinotti et G. Mahuzier dans "Analusis, 1986, v.14, n°6, p. 271-280".

Les marqueurs chimiluminescents actuellement utilisés tels que l'ortophtataldéhyde, la fluorescamine et les dérivés anthracéniques et dansylés, présentent certains inconvénients. Ainsi l'orthophtalaldéhyde et la fluorescamine sont peu sensibles. Les dérivés anthracéniques sont inhibés par l'oxygène dissous et les dérivés dansylés sont inhibés par certains produits de la réaction d'excitation. De plus, certains de ces marqueurs chimiluminescents présentent une toxicité non négligeable, c'est le cas en particulier des dérivés anthracéniques et des dérivés dansylés.

Dans le document précité "Analusis, 1986, v. 14, n° 6, p. 271-280, on a indiqué qu'une coumarine, l'ester éthylique de l'acide diméthylamino-7-coumarinyl-4-acétique présentait des propriétés de fluorescence et de chimiluminescence.

Trois autres coumarines ont été étudiées par Grayeski et al dans Anal. Chem. 1987, 59, 1203-1206 en vue d'une détection par chimiluminescence, mais seulement deux d'entre elles peuvent être détectées de cette façon car on n'observe aucune chimiluminescence avec la troisième.

A la suite de recherches récentes, on a découvert que d'autres noyaux coumariniques présentaient un certain intérêt car ce sont des composés ayant une sensibilité équivalente aux produits les plus performants, qui ne présentent ni toxicité, ni sensibilité vis-à-vis des inhibiteurs usuels tels que l'oxygène dissous, les halogénures et les dérivés nitrés. Par ailleurs, ils sont faciles à mettre en oeuvre dans des conditions d'excitation et de chromatographie variées.

La présente invention a précisément B. 9605 MDT pour objet de nouveaux dérivés coumariniques qui sont des dérivés de tétrahydro-2,3,6,7,1H,5H,11H-(1)benzopyrano(6,7,8.ij)quinolizinone-11 répondant à la formule :

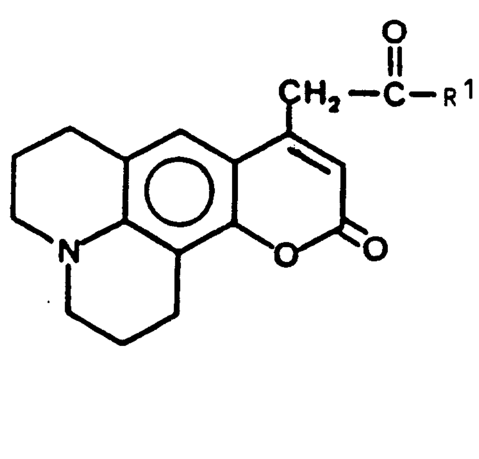

(I)

dans laquelle $R^1$ représente le radical de formule :

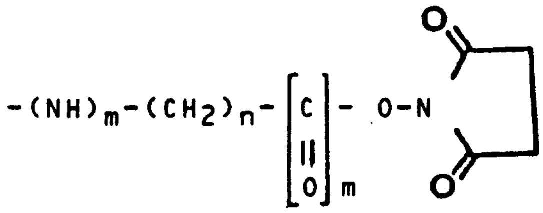

dans laquelle m est égal à 0 ou à 1, et n est égal à 0 ou est un nombre entier allant de 1 à 12 à condition que n soit égal à 0 lorsque m est égal à 0, ou le radical de formule :

$-NH-(CH_2)_n-NH_2$

dans laquelle n à la signification donnée ci-dessus.

Dans ces nouveaux dérivés, l'utilisation du noyau coumarinique de formule :

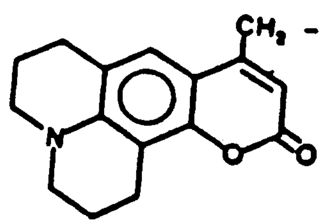

permet d'obtenir des propriétés de fluorescence et de chimiluminescence améliorées ; par ailleurs, le choix d'un groupement $-CO-R^1$ approprié fixé sur ce noyau coumarinique, permet d'obtenir des marqueurs susceptibles d'être utilisés pour un grand nombre de composés organiques.

Ainsi, les nouveaux dérivés de l'invention ont l'avantage d'avoir une bonne sensibilité, de pouvoir être utilisés comme marqueurs pour différents composés, d'être utilisables en phase normale ou inversée, de ne pas être inhibés par les quenchers usuels tels que l'oxygène dissous, les halogénures et les nitrates, de ne pas présenter de toxicité et de réagir rapidement avec les composés à marquer dans des conditions relativement douces.

Le document "Optics Communications, vol. 15, n° 3, novembre / décembre 1975, pages 399-403", étudie les propriétés laser de coumarines ayant différents noyaux de base, parmi lesquelles la coumarine 217 comporte le noyau coumarinique de base des dérivés de l'invention mais avec des substituants différents. Par ailleurs, ce document n'indique pas que la coumarine 217 peut avoir des propriétés de chimiluminescence et il ne suggère pas davantage les performances de ce noyau coumarinique en chimiluminescence.

Selon un premier mode de réalisation de l'invention, le radical $R^1$ représente le radical :

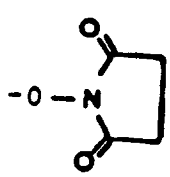

Ce dérivé est la tétrahydro-2,3,6,7,1H,5H,11H(1)-benzopyrano(6,7,8-ij) succinimidoxycarbonylméthyl-9 quinolizinone-11, dénommée ci-après luminarine-1, qui répond à la formule :

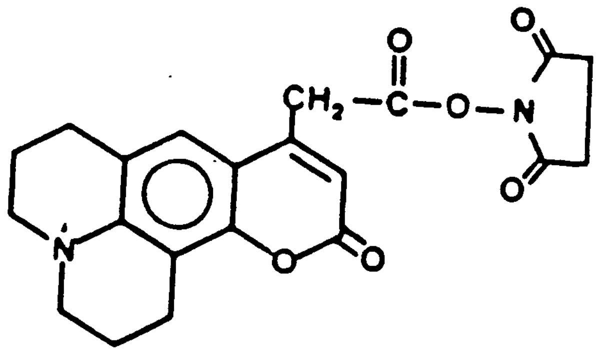

(II)

Ce dérivé est très intéressant car il peut réagir avec les composés comportant des fonctions amine primaire ou secondaire, et le dérivé formé après cette réaction peut être chromatographié en phase liquide et détecté par absorptiométrie, par fluorimétrie ou par chimiluminescence. Dans ce dernier cas qui correspond à la sensibilité maximale, la réaction d'excitation peut utiliser un ester oxalique et du peroxyde d'hydrogène qui forment le peroxyoxalate, intermédiaire réactionnel à haute énergie, qui transfère son énergie au dérivé de formule (II) qui se désexcite en émettant de la lumière. Ce dosage par chimiluminescence peut être appliqué à des molécules d'intérêt biochimique ou pharmacologique en vue d'études de métabolisme ou de pharmacocinétique, notamment aux dosages des acides aminés, des amines biogènes du type histamine, des neuromédiateurs du type catécholamine, des polypeptides hormonaux et des métabolites des phénothiazines, des imipraminiques Ce dérivé de formule (II) trouve donc de nombreuses utilisations.

Selon un second mode de réalisation de l'invention, $R^1$ représente le radical de formule :

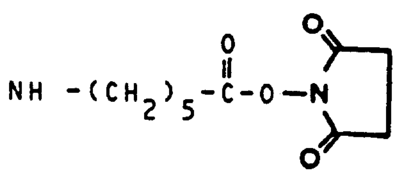

Dans ce cas, le dérivé coumarinique répond à la formule :

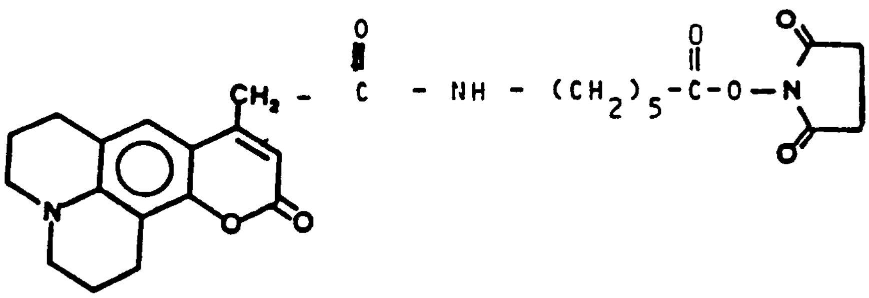

(III)

Ce dérivé dénommé ci-après luminarine-2, peut réagir comme la luminarine-1 avec des composés comportant des fonctions amine primaire ou secondaire. Le dérivé formé peut être chromatographié et détecté par absorptiométrie, fluorimétrie, ou chimiluminescence. Ainsi, on peut utiliser la luminarine-2 pour le dosage des amines biogènes du type histamine et hydroxyéthyl histamine en milieu aqueux.

Selon un troisième mode de réalisation de l'invention, $R^1$ représente le radical de formule $-NH-NH_2$. Ce dérivé coumarinique, dénommé ci-après luminarine-3, répond à la formule :

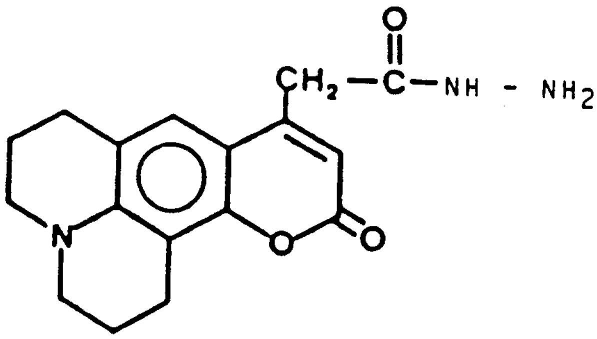

(IV)

Il peut réagir avec les cétones et les aldéhydes et peut de ce fait être utilisé pour le dosage des stéroïdes en milieu polaire, par exemple en milieu hydroalcoolique.

Selon un quatrième mode de réalisation de l'invention, le radical $R^1$ répond à la formule :

$-NH-(CH_2)_4-NH_2$

Le dérivé coumarinique correspondant dénommé ci-après luminarine-4, répond à la formule :

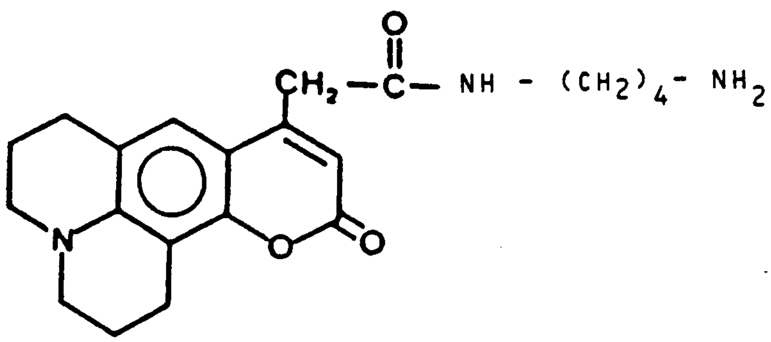

(V)

Ce dérivé peut réagir avec les acides carboxyliques (ou les anhydrides correspondants) et il convient de ce fait à la détection des acides en milieu polaire, par exemple en milieu alcoolique. A titre d'exemple, on peut l'utiliser pour le dosage de l'acide méthylimidazolacétique.

Les nouveaux dérivés coumariniques de l'invention peuvent être préparés par des procédés classiques.

Ainsi, on peut préparer le dérivé de formule (II) par un procédé comprenant les étapes successives suivantes :

1°) faire réagir la 8-hydroxyjulolidine de formule :

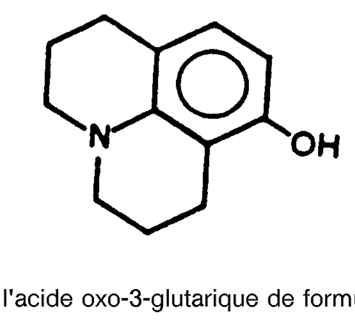

(VI)

avec un ester alkylique de l'acide oxo-3-glutarique de formule

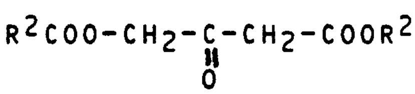

dans laquelle $R^2$ est un radical alkyle, pour former un composé de formule :

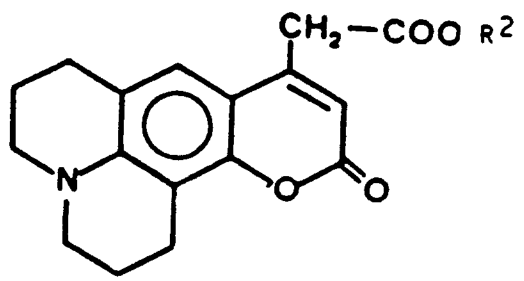

(VII)

2°) hydrolyser l'ester alkylique de formule (VII) obtenu précédemment pour obtenir l'acide de formule :

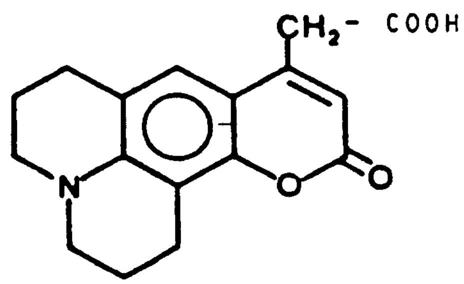

(VIII)

3°) faire réagir l'acide de formule (VIII) ainsi obtenu avec l'oxalate de dihydroxysuccinimide de formule :

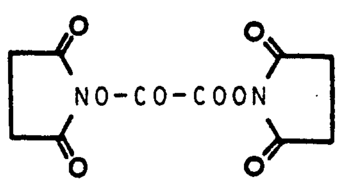

(IX)

Dans ce procédé, la première étape peut être effectuée en portant au reflux sous agitation la 8-hydroxyjulolidine avec l'ester alkylique de l'acide oxo-3-glutarique, en présence de chlorure de zinc anhydre, et en solution alcoolique. Le composé de formule (VII) obtenu peut être séparé par extraction au moyen d'un solvant organique tel que l'acétate d'éthyle.

Dans la deuxième étape du procédé, on peut réaliser l'hydrolyse de l'ester alkylique de formule (VII) par une solution aqueuse de soude dans une solution alcoolique constituée par exemple de méthanol.

La troisième étape du procédé peut être réalisée par exemple en faisant réagir l'acide de formule (VIII) avec l'oxalate de dihydroxysuccinimide dans un solvant organique anhydre, en présence de triéthylamine anhydre.Dans ces conditions, on peut obtenir le dérivé coumarinique de formule (II) avec un degré de pureté élevé, d'environ 90%.

Les dérivés coumariniques répondant à la formule

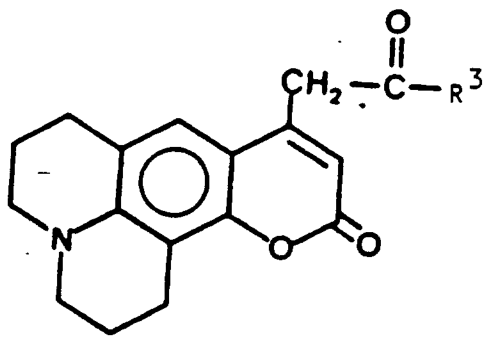

(X)

dans laquelle $R^3$ représente le radical de formule

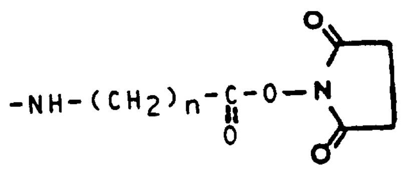

dans laquelle n est un nombre entier allant de 1 à 12, peuvent être préparés par un procédé comprenant les étapes successives suivantes :

a) faire réagir le composé de formule :

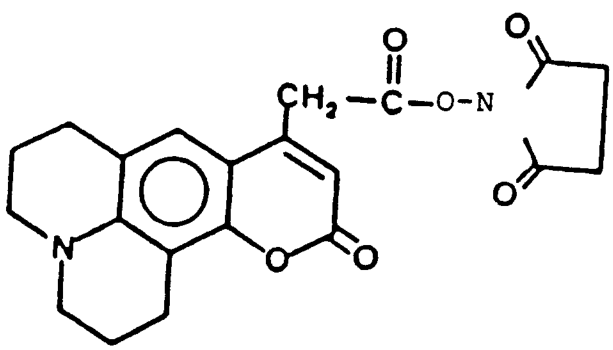

(II)

avec un aminocarboxylate d'alkyle de formule :

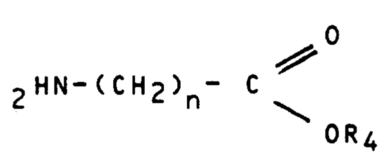

dans laquelle $R^4$ est un radical alkyle de 1 à 4 atomes de carbone et n a la signification donnée ci-dessus, pour former un composé de formule :

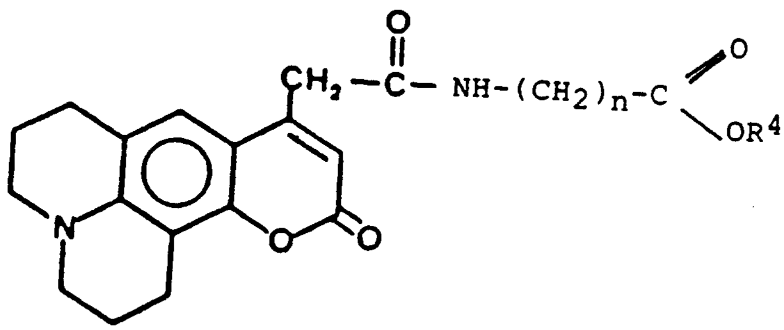

(XIII)

b) hydrolyser l'ester de formule (XIII) obtenu, pour former l'acide de formule :

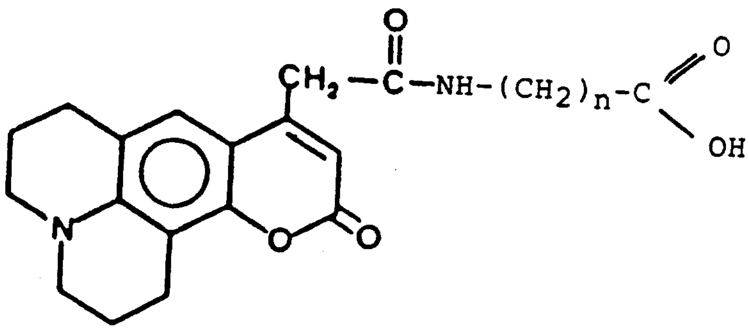

(XIV)

c) faire réagir l'acide de formule (XIV) ainsi obtenu avec

1) le carbonate de dihydroxysuccinimide de formule :

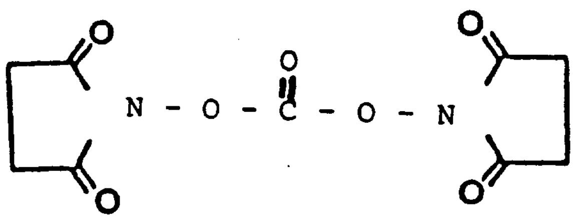

(XV)

2) l'oxalate de dihydroxysuccinimide, ou

3) l'hydroxysuccinimide en présence de dicyclohexylcarbodiimide.

A titre d'exemple, lorsque n est égal à 5, on peut faire réagir la luminarine-1 de formule (II) avec l'amino-6 hexanoate de méthyle, puis hydrolyser l'ester obtenu en acide et transformer l'acide en ester d'hydroxy succinimide par action de carbonate ou d'oxalate d'hydroxysuccinimide, ou par action d'hydroxysuccinimide en présence de dicyclohexylcarbodiimide.

Les dérivés coumariniques répondant à la formule (X) dans laquelle $R^3$ est le radical de formule $-NH-(CH_2)_n-NH_2$ dans laquelle n est un nombre entier allant de 1 à 12, peuvent être préparés par un procédé consistant à faire réagir le composé de formule :

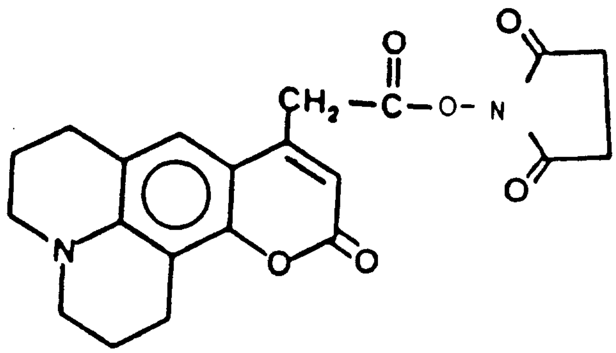

(II)

avec un diaminoalcane de formule

$_2HN-(CH_2)_n-NH_2$

dans laquelle n a la signification donnée ci-dessus.

A titre d'exemple, lorsque n est égal à 4, on peut faire réagir l'ester méthylique de formule (VII) sur la butane diamine-1,4.

Le dérivé coumarinique répondant à la formule :

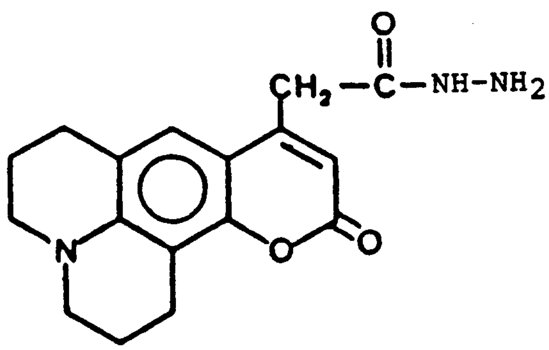

(IV)

peut être préparé par le procédé comprenant les étapes successives suivantes :

1°) faire réagir la 8-hydroxyjulolidine de formule :

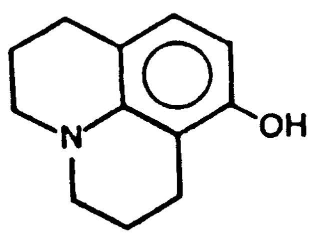

(VI)

avec un ester alkylique de l'acide oxo-3-glutarique de formule :

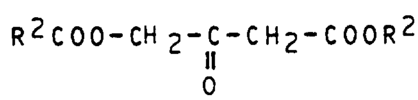

dans laquelle $R^2$ est un radical alkyle, pour former un composé de formule :

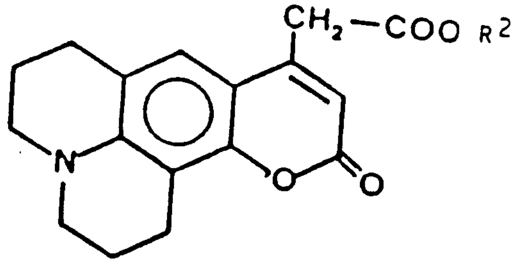

(VII)

2°) faire réagir l'ester alkylique de formule (VII) ainsi obtenu avec l'hydrate d'hydrazine $_2HN\text{-}NH_2$, $H_2O$.

L'ester de formule (VII) peut être en particulier l'ester méthylique.

Dans les procédés décrits ci-dessus pour la préparation de dérivés coumariniques de formule (II) ou de formule (X), l'ester alkylique de l'acide oxo-3-glutarique est un ester dans lequel le groupe alkyle a de préférence de 1 à 2 atones de carbone. A titre d'exemples, on peut utiliser l'ester éthylique.

Les produits de départ utilisés dans ces différents modes de préparation des dérivés coumariniques de l'invention sont des produits du commerce ou peuvent être préparés par des procédés classiques à partir de produits du commerce.

Comme on l'a indiqué précédemment, les dérivés coumariniques de l'invention peuvent réagir avec différents composés organiques, par exemple des amines dans le cas de la luminarine-1 et de luminarine-2, des aldéhydes et des cétones dans le cas de la luminarine-3, et des acides carboxyliques dans le cas de la luminarine-4.

Aussi, ces dérivés coumariniques peuvent être utilisés comme marqueurs de composés organiques comportant des fonctions amine primaire ou secondaire, aldéhyde, cétone, ou acide carboxylique, et servir à la détection de ces composés dans des méthodes de dosage par chromatographie en phase liquide. La détection peut être effectuée par absorptiométrie, fluorimétrie ou chimiluminescence, en utilisant les méthodes classiques telles que celles décrites par M.Tsitini Tsamis et al dans Journal of chromatography, 277,(1983), 61-69 ; par D. Amir et E. Haas dans Int. J. Peptide Protein Res. 26, 1986, p.7-17 ; par H. Cisse et al dans Journal of Chromatography, 225, (1981), p. 509-515 et par M. Tod et al dans Analusis, 1986, v14, n°6 p. 271-280.

Aussi, l'invention a également pour objet un procédé de détection d'un composé comportant une fonction amine primaire ou secondaire par chromatographie en phase liquide, qui consiste à faire réagir le composé à détecter avec un dérivé répondant à la formule :

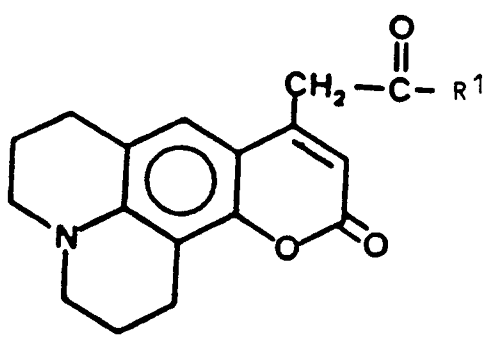

(I)

dans laquelle $R^1$ représente le radical de formule :

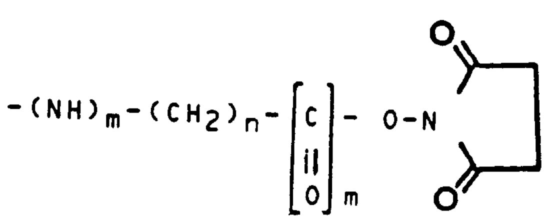

dans laquelle m = 0 ou 1 et n = 0 ou est un nombre entier allant de 1 à 12 à condition que n = 0 lorsque m = 0, pour former un dérivé du composé à détecter, à réaliser ensuite une séparation de ce dérivé par chromatographie en phase liquide et à détecter ensuite ce dérivé par absorptiométrie, fluorimétrie, ou chimiluminescence.

De préférence, on réalise la détection par chimiluminescence en utilisant un ester oxalique et du peroxyde d'hydrogène.

A titre d'exemple, l'ester oxalique peut être le bis-(trichloro-2,4,6 phényl)oxalate (TCPO)ou le bis-(dinitro-2,4 phényl)oxalate (DNPO).

L'invention sera mieux comprise à la lecture des exemples suivants, donnés bien entendu à titre illustratif et non limitatif, en référence au dessin annexé sur lequel :

- la figure 1 représente le spectre de masse de la luminarine-1,
- la figure 2 représente le spectre en résonance magnétique nucléaire du proton de la luminarine-1,
- la figure 3 représente le spectre infrarouge de la luminarine-1,
- la figure 4 représente le spectre de masse du dérivé obtenu par réaction de la pentylamine avec la luminarine-1,
- la figure 5 représente le spectre de masse du dérivé obtenu par réaction de la dipropylamine avec la luminarine-1,
- la figure 6 représente le spectre de masse de la luminarine-2,
- la figure 7 représente le spectre de masse de la luminarine-3,
- la figure 8 représente le spectre en résonance magnétique nucléaire du proton de la luminarine-3,
- la figure 9 représente le spectre de masse de la luminarine-4, et
- la figure 10 représente le spectre en résonance magnétique nucléaire du proton de la luminarine-4.

**Exemple 1** : Synthèse de la luminarine-1 répondant à la formule :

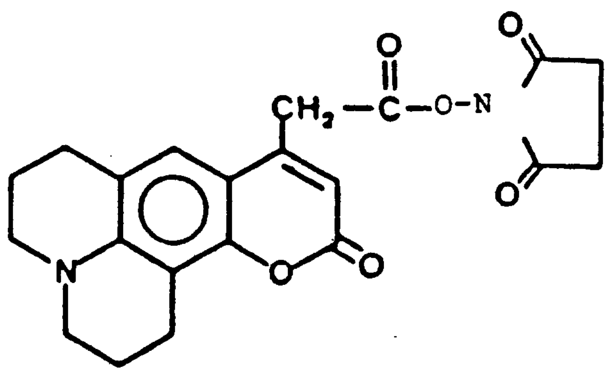

(II)

a) préparation du composé de formule (VII) avec $R^2$ représentant le radical éthyle.

La réaction effectuée ici correspond au schéma réactionnel suivant :

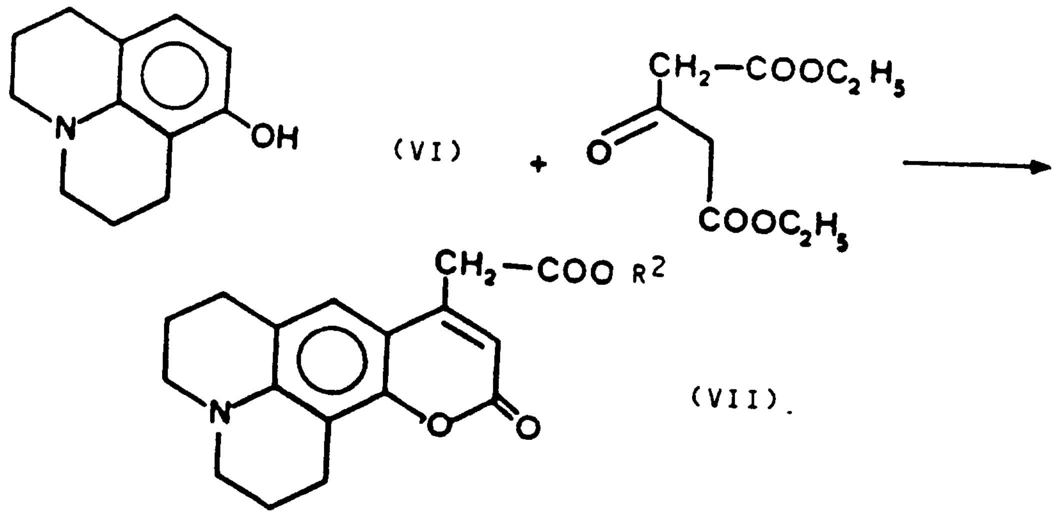

(VI) + 

(VII).

On introduit dans un récipient 2,12g de 8-hydroxyjulolidine, 2,22g d'ester éthylique de l'acide oxo-3-glutarique, 1,71g de chlorure de zinc anhydre et 6ml d'éthanol anhydre, et on porte au reflux pendant 24h, sous agitation à l'abri de l'humidité. Après refroidissement, on introduit la solution dans 200ml d'eau, puis on extrait par 200ml, puis 100ml d'acétate d'éthyle. On lave la phase organique à l'eau, on la sèche sur sulfate de magnésium et on la concentre à sec. On recristallise ensuite dans 5 parties d'acétate d'éthyle. On obtient ainsi l'ester éthylique de formule (VII) avec un rendement de 56%.

b) Hydrolyse de l'ester éthylique de formule (VII) obtenu précédemment.

On introduit dans un récipient 2g de l'ester éthylique obtenu précédemment avec 42ml d'une solution aqueuse de NaOH à 1,2% P/V et 40ml de méthanol, puis on porte à 45 °C pendant une heure. Après refroidissement, on extrait le milieu réactionnel par 50ml, puis 40ml de chloroforme. Après dégazage, on acidifie la phase aqueuse avec 16ml d'acide chlorhydrique 3N, puis on maintient le mélange réactionnel sous agitation pendant 15min. On ajuste alors le pH à 6,5 avec 13ml de soude 2,5N. On filtre alors le précipité formé, on le rince à l'eau et on le sèche. On obtient ainsi l'acide de formule (VIII) avec un rendement de 90%.

c) Obtention de la luminarine-1.

Dans cette troisième étape, on fait réagir l'acide de formule (VIII) obtenu dans l'étape précédente avec l'oxalate de dihydroxysuccinimide selon le schéma réactionnel suivant :

2 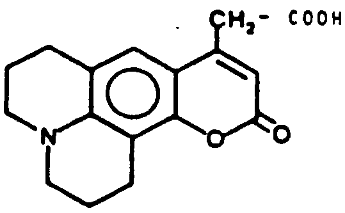 (VIII) + 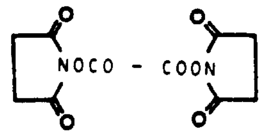 (IX)

2 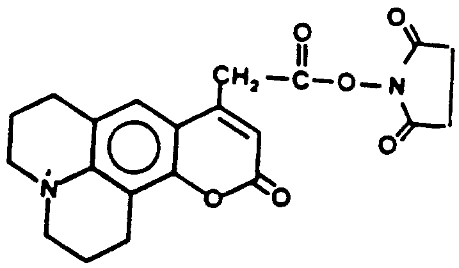 (II) + $HOOC - COOH$

On introduit dans un récipient 1,5g de l'acide de formule (VIII), 2,13g d'oxalate de dihydroxysuccinimide, 0,51g de triéthylamine anhydre et 220ml d'acétonitrile anhydre. On maintient le mélange sous agitation, à l'abri de l'humidité, pendant une heure à la température ambiante, puis pendant une heure à une température de 35-40 °C. On filtre alors un léger insoluble, puis on concentre sous vide et on purifie par chromatographie sur gel de silice en utilisant comme solvant d'élution le mélange dichlorométhane / tétrahydrofuranne (THF) avec un rapport en volume de 1/1. On concentre les fractions 11 à 14, et l'on obtient ainsi la luminarine-1 avec un rendement de 21%.

On vérifie la structure de la luminarine-1 par les méthodes suivantes :

- 1) <u>détermination de la formule brute</u> :

| $C_{21}H_{20}O_6N_2$ | | | | |
|---|---|---|---|---|
| | C | H | N | O |
| Calculé<br>Trouvé | 0,64<br>0,635 | 0,051<br>0,051 | 0,071<br>0,070 | 0,24<br>0,24 |

- 2) <u>Spectrométrie de masse en impact électronique à 70eV avec introduction directe.</u>

Le spectre obtenu est représenté sur la figure 1, et on y trouve le pic moléculaire à 396 et les principaux fragments à 298, 282, 254 et 226, ce qui correspond à la structure suivante :

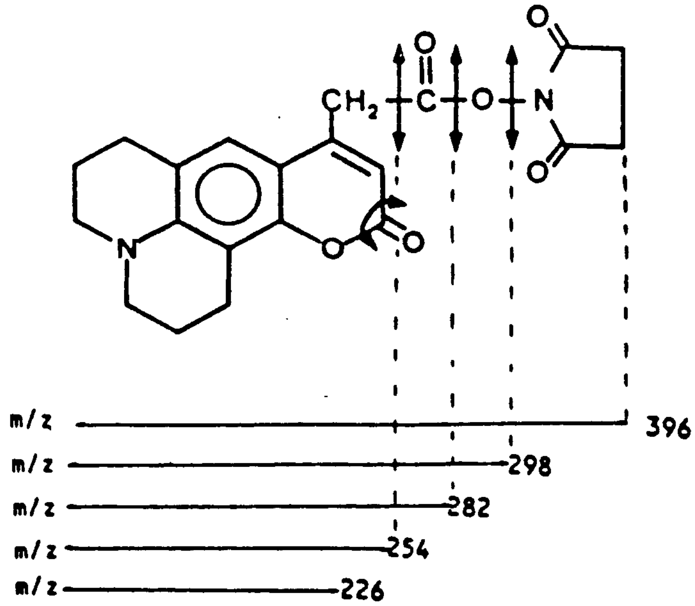


- 3) Spectrométrie par résonance magnétique nucléaire (RMN).

Le spectre RMN du proton de la luminarine-1 dans l'acétone deutérié est représenté sur la figure 2. Il correspond aux résultats suivants :

| DEPLACEMENT (ppm) | NATURE | HAUTEUR | ORIGINE |
|---|---|---|---|
| 1,94 | Multiplet | 4 H | $CH_2-CH_2-\bar{N}-CH_2-CH_2$ |
| 2,78 | Sextuplet | 4 H | $CH_2-N-CH_2$ |
| 2,88 | Singulet | 4 H | Succinimide |
| 3,29 | Multiplet | 4 H | $CH_2$ en 6 et 8 |
| 4,18 | Singulet | 2 H | $-CH_2-\overset{O}{\overset{\|}{C}}-O$ |
| 6,11 | Singulet | 1 H | $=C-H$ en 3 |
| 7,13 | Singulet | 1 H | H en 5 |

- 4) Spectrométrie infrarouge.

Le spectre infrarouge de la luminarine-1 en pastille de KBr est représenté sur la figure 3. Il correspond aux résultats suivants :

| Nombre d'onde cm-1 | Transmission % | Origine |
|---|---|---|
| 2940 | 53 | Val. asymétrique CH3 |
| 2840 | 58 | Val. symétrique CH3 |
| 1800 | 45 | -------------------- |
| 1770 | 34 | Val. C = O de succinimide |
| 1730 | 3 | Val. C = O de pyrone |
| 1700 | 8 | Val. C = O de l'ester |
| 1590 | 11 | |
| 1550 | 35 | Val. C = C aromatique |
| 1510 | 39 | |
| 1420 | 32 | -------------------- |
| 1370 | 24 | -------------------- |
| 1310 | 23 | Val, C-N aromatique |
| 1260 | 54 | Val. C-O-C pyrone |
| 1200 | 20 | -------------------- |
| 1090 | 20 | -------------------- |
| 990,920 | 72 | Déf C-H aromatique |
| 840,800,655 | 68,69,68 | -------------------- |

On détermine également la pureté de la luminarine-1 par chromatographie liquide sous haute pression et détection fluorimétrique. Cette pureté est de 90%. On observe par ailleurs que la principale impureté est constituée par l'acide de formule (VIII). Une analyse par chromatographie en phase gazeuse et spectrométrie de masse permet d'identifier en plus de l'acide de formule (VIII), d'autres inpuretés en petites quantités qui sont l'ester éthylique de formule (VII) et le produit de décarboxylation de l'acide de formule (VII).

La luminarine-1 est une poudre jaune cristalline que l'on peut conserver en flacon brun à l'abri de la lumière, et que l'on peut mettre en solution dans le tétrahydrofuranne, l'acétone ou le méthanol, sa solubilité dans ces solvants organiques étant d'environ 1g/l.

La stabilité d'une solution à $2.10^{-3}$mol/l de luminarine-1 dans le tétrahydrofuranne a été étudiée par chromatographie liquide sous haute pression et détection fluorimétrique. On constate ainsi que le produit en solution se dégrade de façon significative en quelques heures à la température ambiante par hydrolyse, avec formation de l'acide de formule (VIII). En revanche, la solution congelée se conserve au moins deux mois sans dégradation apparente.

**Exemple 2** : Réaction de la luminarine-1 avec la pentylamine.

Cette réaction correspond au schéma réactionnel suivant.

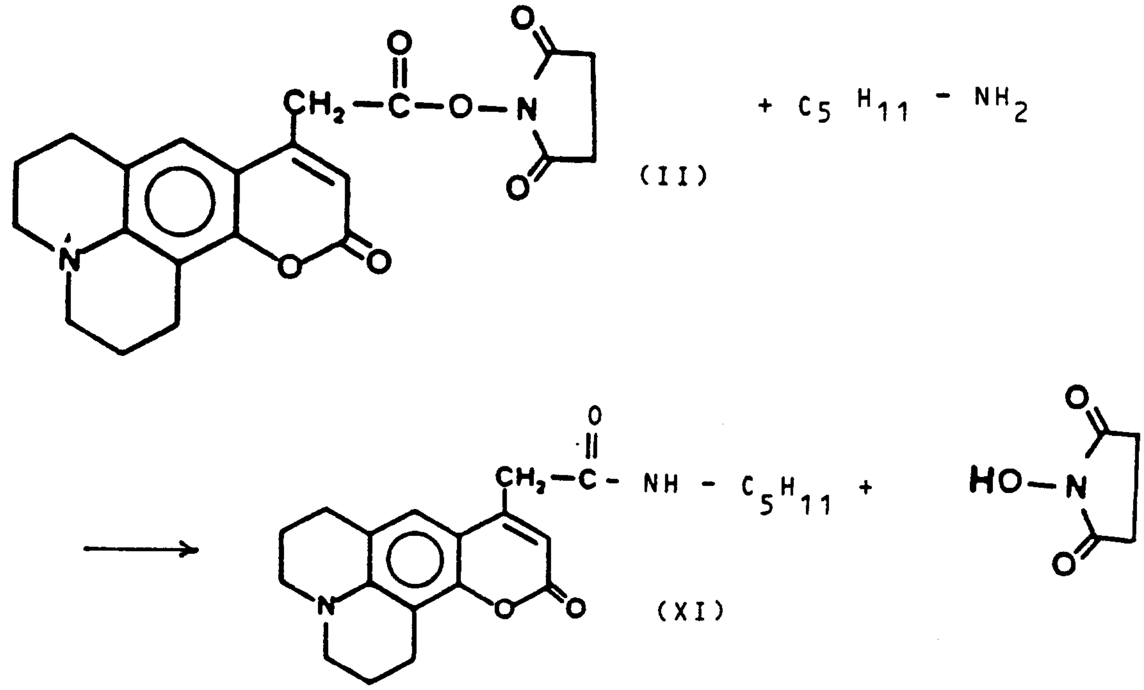

(II)

(XI)

On fait réagir 4g de luminarine-1 avec 0,9g de pentylamine dans 2000ml de THF à 50 °C pendant 20min. On obtient ainsi le dérivé de la pentylamine de formule (XI) avec un rendement de 97%. La structure de ce dérivé a été confirmée par chromatographie en phase gazeuse et spectrométrie de masse.

La figure 4 représente le spectre de masse du dérivé de la pentylamine et de la luminarine de formule (XI).

**Exemple 3** : Réaction de la luminarine-1 avec la dipropylamine.

La réaction est effectuée comme dans l'exemple 2 en faisant réagir 4g de luminarine avec 0,9g de dipropylamine dans 2000ml de THF, à 80 °C pendant 30min, en présence de pyridine comme catalyseur. On analyse le produit obtenu par chromatographie en phase gazeuse et spectrométrie de masse. Le spectre de masse est représenté sur la figure 5 et confirme la structure correspondant à la formule ci-dessous :

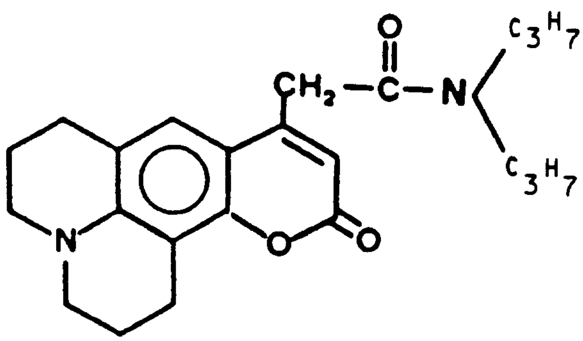

(XII)

**Exemple 4** : Utilisation de la luminarine-1 pour détecter la pentylamine par chromatographie en phase liquide.

Dans cet exemple, on soumet à une chromatographie liquide le dérivé de la pentylamine obtenu dans l'exemple 2.

L'installation de chromatographie liquide comprend une pompe Chromatem 380, un amortisseur de pulsations, un injecteur Rhéodyne de 20µl, une colonne de silice et un détecteur fluorimétrique Schoeffel FS 970 avec un enregistreur Servotrace. Ce système est complété par un mélangeur post-colonne Kratos UFR 051 de 500µl et un four à colonne Shimadzu lorsqu'on réalise la détection par chimiluminescence.

On utilise comme phase stationnaire une colonne de silice greffée octadécyle licrosphère de 300x4,6mm avec une dimension moyenne des particules de 5µm.

La phase liquide mobile est un mélange d'acétonitrile et d'eau avec un rapport en volume acétonitrile / eau de 70/30. On introduit cette phase liquide à un débit de 1ml/min et on y ajoute la luminarine-1 pure ou le dérivé de luminarine-1 et de pentylamine. On détecte les produits obtenus en sortie de colonne par fluorescence en réalisant l'excitation à 380nm et en détectant l'émission avec un filtre passe haut à 470nm.

Dans ces conditions la luminarine-1 est détectée 3,2min après l'injection, ce qui correspond à un k' de 2,2, et le dérivé de luminarine-1 et de pentylamine est détecté 3,8min après l'injection, ce qui correspond à un k' de 2,8.

Dans une deuxième expérience, on détecte les produits en sortie de colonne par chimiluminescence en utilisant le bis(trichloro-2,4,6 phényl)-oxalate (TCPO) et $H_2O_2$. Dans ce cas, on utilise comme phase stationnaire une colonne de silice greffée octadécyle licrosphère de 150x4,6mm avec une dimension moyenne de particules de 3µm. La phase mobile est un mélange d'acétonitrile et de tampon imidazole-nitrate à pH 8 et à $10^{-2}$mol/l, avec un rapport en volume acétonitrile/tampon de 70/30, et un débit de 1ml/min. La réaction de chimiluminescence est effectuée en utilisant une solution à $10^{-2}$mol/l de TCPO dans de l'acétate de méthyle, à un débit de 0,25ml/min et une solution à 0,2mol/l de $H_2O_2$ dans le tétrahydrofuranne, à un débit de 0,25ml/min. On utilise le même filtre passe haut à 470nm et un four de colonne maintenu à 30 °C.

Dans ces conditions, le dérivé de pentylamine et de luminarine sort après 1,4min, ce qui correspond à un k' de 3,5.

Dans une troisième expérience, on détecte les produits en sortie de colonne par chimiluminescence en utilisant le DNPO et $H_2O_2$. Dans ce cas la phase solide utilisée est une colonne de silice de 150x4,6mm avec une dimension moyenne de particules de 5µm. La phase liquide mobile est un mélange de chloroforme, d'hexane, d'acétate de méthyle et d'acide acétique dans un rapport en volume de 66/17/17/0,1, avec un débit de 2ml/min. Pour la réaction de chimiluminescence, on utilise une solution à 0.4mol/l de $H_2O_2$ dans l'acétonitrile à un débit de 0,50ml/min et une solution à 0,006mol/l de bis-(dinitro-2,4 phényl)oxalate (DNPO) dans l'acétate de méthyle à un débit de 0,17ml/min. La détection est réalisée avec le filtre passe-haut à 470nm.

Dans ces conditions, le dérivé de pentylamine et de luminarine sort à 2,4min, ce qui correspond à un k' de 3,0.

On précise que la limite de détection du dérivé de pentylamine et de luminarine en chromatographie liquide avec détection fluorimétrique est de 380fmol, et qu'elle est considérablement améliorée avec la détection par chimiluminescence où elle passe à 2fmol injectées, la réponse étant linéaire entre 2 et 500fmol injectées.

A titre d'exemple, on donne dans le tableau 1 annexé, les résultats obtenus dans les mêmes conditions avec d'autres marqueurs chimiluminescents.

Au vu de ce tableau 1, on constate que la limite de détection la plus faible est obtenue avec la luminarine-1 en chimiluminescence.

**Exemple 5** : Synthèse de la luminarine-2 de formule :

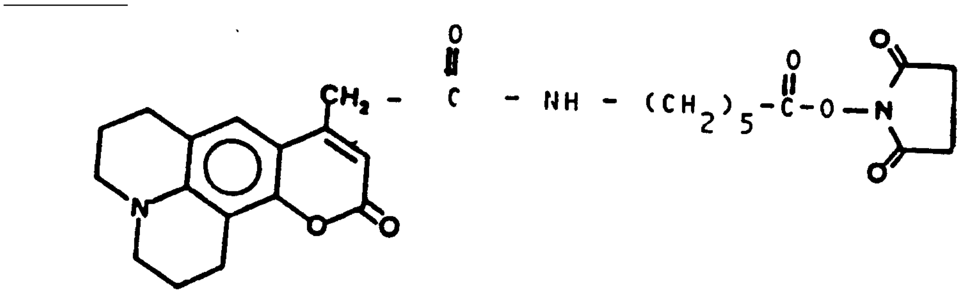

(III)

On part de la luminarine-1 (II) décrite dans l'exemple 1, et on réalise les étapes suivantes :

a) action de l'aminohexanoate de méthyle sur la luminarine-1 :

La réaction correspond au schéma réactionnel suivant :

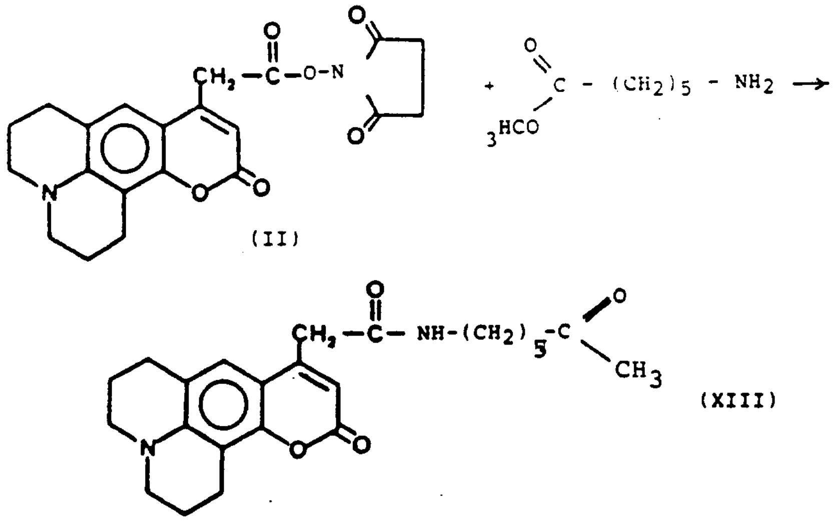

(II)

(XIII)

On prépare de la luminarine-1 (II)par action de 11,26g (0,0377 mol ) d'acide (VIII), 3,81g de triéthylamine (0,0377 mol ) et 10,62g de di-NN' succinimidyle carbonate (0,0415 mol ) dans 560ml d'acétonitrile sec (solution A).

On fait réagir 30g de carbonate de potassium sur une solution de 30g de chlorhydrate d'aminohexanoate de méthyle (solution B) ; On filtre après 30 heures.

On mélange les solutions A et B et on agite durant 8 heures.

On ajoute 20g d'éthanolamine et on agite une demi-heure.

On filtre, lave à l'eau, sèche le solvant et met à sec.

Le résidu d'évaporation est purifié par chromatographie sur colonne de silice (solvant : $CH_2Cl_2$, $CH_2Cl_2$-THF 85 : 15, puis $CH_2Cl_2$-THF 75-25).

Les fractions enrichies sont recristallisées dans l'acétate d'éthyle.

Rendement 5,76g (36%).

b) Hydrolyse de l'ester éthylique (XIII) obtenu : à partir de 1,25g de l'ester (XIII), on obtient 0,76g de l'acide (XIV) en opérant exactement comme dans l'exemple 1 b)(Rt : 63%).

c) Obtention de la luminarine-2 (III), ester d'hydroxysuccinimide de l'acide (XIV) :

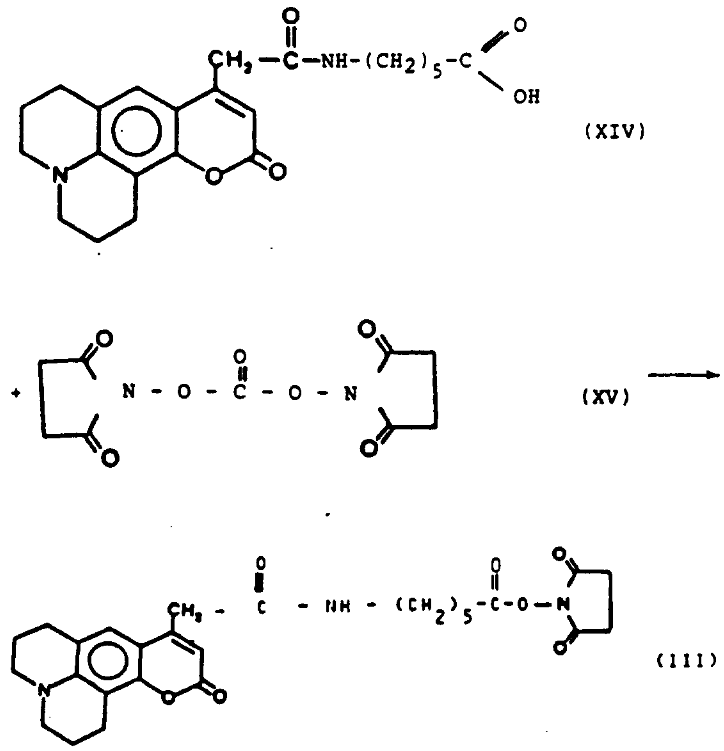

(XIV)

(XV)

(III)

On fait réagir, durant 6 heures, 0,750g d'acide (XIV), 0,912g de triéthylamine, 0,512g de carbonate de dihydroxysuccinimide (XV) dans 27ml d'acétonitrile.

Après filtration et évaporation, on purifie par chromatographie sur colonne de silice. (Solvant : $CH_2Cl_2$, $CH_2Cl_2$-THF 85 : 15 puis $CH_2Cl_2$-THF 75 : 25).

Rendement des fractions pures : 175mg (19%).

On obtient ainsi la luminarine-2 et l'on vérifie par spectrométrie de masse que sa structure correspond à la formule donnée ci-dessus.

Le spectre de masse est représenté sur la figure 6 et confirme bien cette structure.

**Exemple 6** : Synthèse de la luminarine-3 de formule :

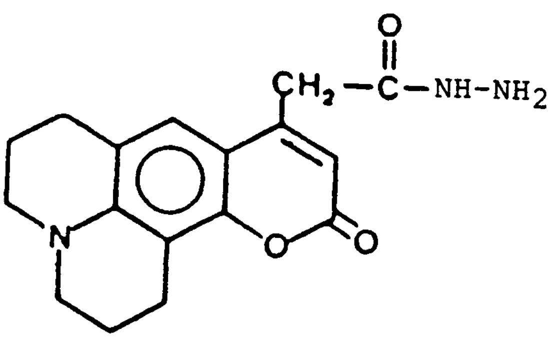

(IV)

On part de l'ester (VII) ($R_2 = CH_3$) décrit dans l'exemple 1 . a) que l'on fait réagir sur l'hydrate d'hydrazine, selon le schéma suivant :

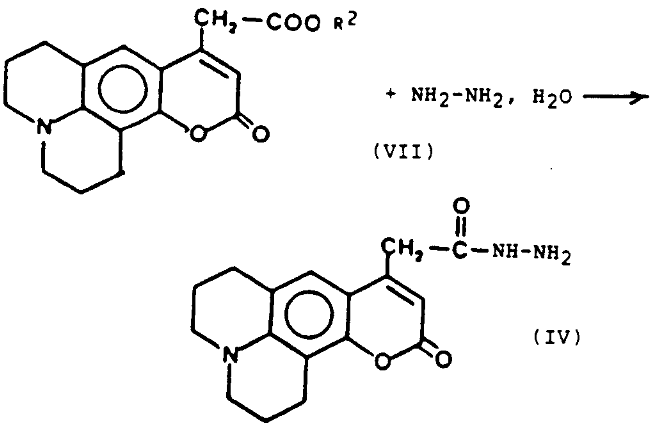


(VII)

(IV)

On fait réagir durant 4 heures, sous agitation, 5g d'ester (VII) (15 mmoles) et 8ml d'hydrazine (150mmoles), dans 100ml de méthanol.

Après 4 heures, on filtre, rince avec 10ml de méthanol, puis avec 10ml de dichlorométhane.

Rendement : 3,6g (77%)

On vérifie par spectrométrie de masse et par RMN que le composé obtenu est bien la luminarine-3.

Le spectre de masse est représenté sur la figure 7 et le spectre RMN sur la figure 8 ; ils confirment bien que le composé obtenu correspond à la formule (IV) donnée ci-dessus.

**Exemple 7** : Synthèse de la luminarine-4 de formule :

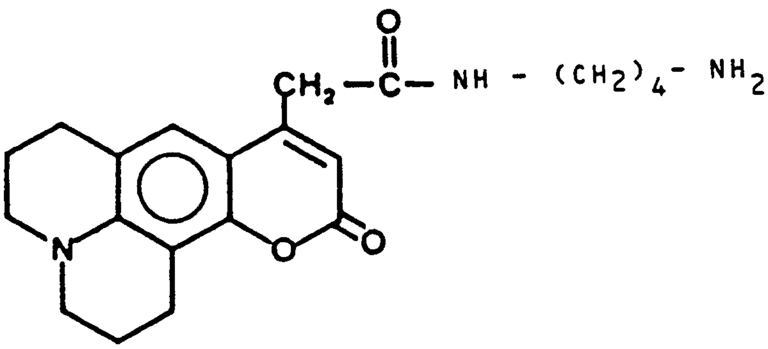

(V)

On suit le méme mode opératoire que dans l'exemple 5 (paragraphe a) sauf que l'on utilise la diamine de formule $NH_2$-$(CH_2)_4$-$NH_2$ au lieu de l'aminohexanoate de méthyle.

On part directement de la luminarine-1 (II) purifiée que l'on fait réagir sur le diamino 1-4 butane selon le schéma réactionnel suivant :

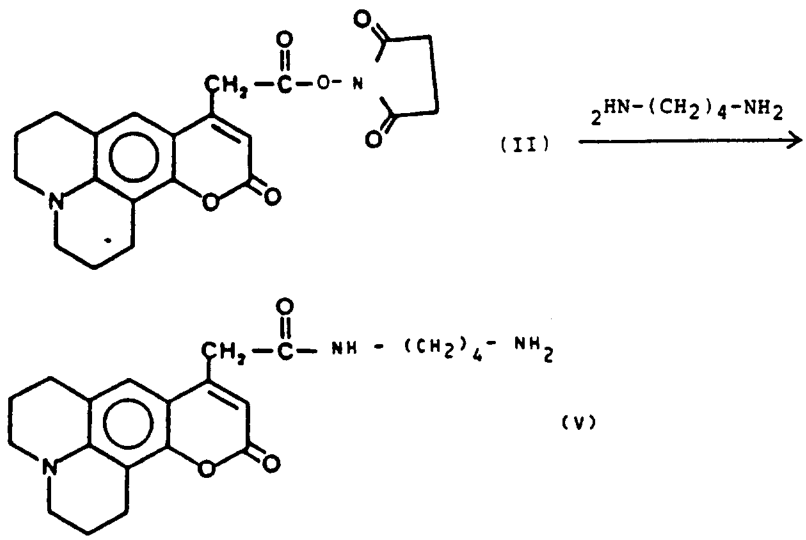


Dans un ballon d'un litre, on fait réagir 16,5g de luminarine-1 (II) (41,6mmoles) et 18,3g de 1-4 diaminobutane (10$^7$mmoles) dans 400ml de THF anhydre, sous agitation durant 24 heures.

On filtre un insoluble marron.

On concentre à sec les eaux mères ; on reprend le résidu dans 100ml de dichlorométhane et on extrait par 5x100ml d'eau pour éliminer l'excès de 1-4 diaminobutane.

On concentre le solvant à sec.

Le résidu est mis sous agitation dans 25ml de dichlorométhane.

Rendement : 10,6g (69%).

On vérifie par spectrométrie de masse et RMN la structure du composé obtenu.

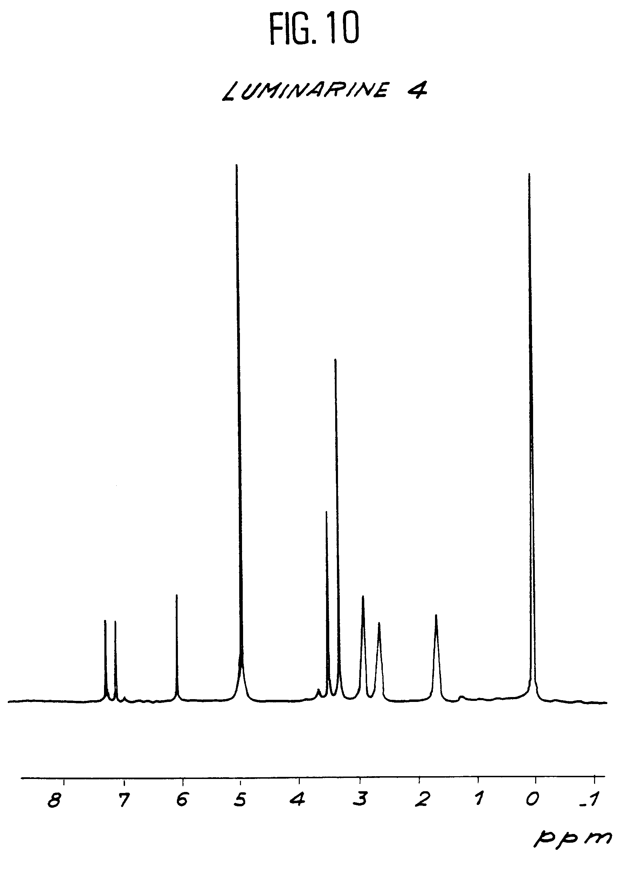

Le spectre de masse est représenté sur la figure 9 et le spectre RMN sur la figure 10 et ils confirment bien que la structure du composé obtenu correspond à la formule (V) donnée ci-dessus.

**Exemple 8.**

Pour mettre en évidence les propriétés améliorées des luminarines de l'invention, on a effectué des mesures de fluorescence, d'absorbance et de chimiluminescence en utilisant comme fluorophore des coumarines de l'art antérieur qui sont la coumarine-1®, diméthylamino-7 méthyl-4 coumarine (DEMC) de formule :

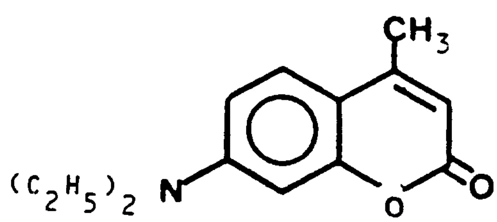

et la coumarine-311®, diméthylamino-7 méthyl-4 coumarine (DMMC) de formule :

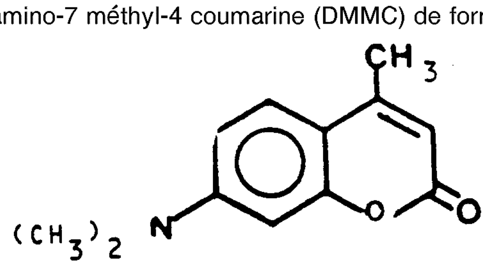

pour les comparer avec la coumarine-102®,tétrahydro-2, 3, 5, 6, 7, 1H, 5H, 11H-(1)- benzopyrano (6, 7, 8-ij) méthyl-9 quinolizinone-11(TBMQ) qui est le noyau d base des dérivés de l'invention de formule :

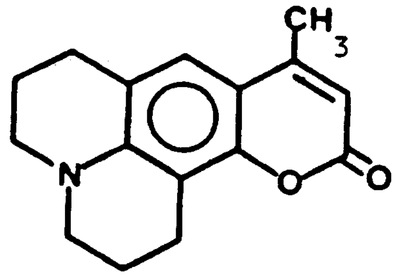

Les mesures de fluorescence sont effectuées avec un spectrofluorimètre Perkin Elmer LS5 avec des largeurs de fentes de 2nm pour les deux monochromateurs.

Les mesures d'absorbance sont effectuées avec un spectrophotomètre Jobin Yvon JY3. Les mesures de chimiluminescence sont obtenues en utilisant un spectrofluorimètre Jobin Yvon JY4 en utilisant des largeurs de fentes de 10nm. La cellule d'échantillons liquides est une cuvette de quartz de 10x10mm d'une capacité de 3ml remplie de 2ml de solution, ce qui correspond à un volume réel de solution exposée au photomultiplicateur de 1,35ml.

**Mesures de fluorescence et d'absorbance.**

Ces mesures ont été effectuées à 20 ± 2°C, et on a déterminé les rendements quantiques de fluorescence $R_F$ des coumarines-1, 311 et 102 par rapport au bisulfate de quinine dont le rendement quantique de fluorescence est de 0,546, en suivant la méthode de Parker décrite dans Analyst, 85,(1960), p. 587, avec une solution de bisulfate de quinine à 2mg/l dans $H_2SO_4$ 0,1N.

Pour les coumarines, on a utilisé une solution d'acétone et d'acétate d'éthyle dans un rapport en volume 75/25 avec une concentration en coumarine de $10^{-6}$mol/l en assurant dans chaque cas une absorbance inférieure à 0,05.

**Mesures de chimiluminescence.**

Pour déterminer les rendements quantiques de chimiluminescence $R_C$, on a estimé l'intensité en fonction du temps au maximum de l'émission. Lorsque la décroissance de l'intensité est assez basse, le spectre d'émission est tracé, puis l'intensité en fonction du temps est suivie jusqu'à ce que l'intensité soit tombée à 2% de l'intensité maximale.

Les autres paramètres de luminescence sont mesurés directement, il s'agit de l'intensité maximale (Imax), du temps pour atteindre l'intensité maximale (Tmax), et du temps pour atteindre la moitié de l'intensité maximale après le temps maximal (T1/2).

Le rendement de chimiluminescence $R_C$ est calculé en suivant la méthode de Rauhut décrite dans J. Am. Chem. Soc., 88, 15(1966), 3604.

Le rendement d'excitation $R_{ex}$ est déterminé à partir de la formule :

$$R_C = R_{ex} \times R_F$$

Pour les mesures de chimiluminescence avec le TCPO, on ajoute à 1ml de la solution contenant $10^{-3}$mol/l de coumarine, 0,5ml d'une solution à $2.10^{-3}$mol/l de TCPO dans de l'acétate d'éthyle, 0,5ml d'une solution à $2.10^{-2}$mol/l de $H_2O_2$ dans l'acétone et 10µl d'un tampon imidazole nitrate, pH 7,5, à 0,1mol/l. Lorsqu'on utilise le DNPO, on ajoute à la même solution de coumarine dans l'acétone, 0,5ml d'une solution à $10^{-3}$mol/l de DNPO dans l'acétate d'éthyle et 0,5ml d'une solution à $10^{-2}$ mol/l de $H_2O_2$ dans de l'acétone.

Les propriétés de fluorescence et d'absorbance de ces coumarines sont données dans le tableau 2, et les résultats de chimiluminescence sont donnés dans le tableau 3.

Au vu des tableaux 2 et 3, on remarque que le noyau coumarinique utilisé dans l'invention qui correspond à la coumarine 102, présente les meilleures propriétés de fluorescence, d'absorbance et de chimiluminescence.

Dans le tableau 4, on a donné les résultats obtenus en ce qui concerne le rendement de chimiluminescence $R_C$ en utilisant TCPO et $H_2O_2$ en fonction de la concentration en coumarine-102, ainsi que les valeurs de Imax, Tmax et T1/2.

Au vu de ces résultats, on constate que le rendement de chimiluminescence augmente avec la concentration en coumarine-102.

Dans le tableau 5, on a donné les variations du rendement de chimiluminescence, de Imax, de Tmax et de T1/2, en fonction du pH de la solution.

Les résultats obtenus montrent que le rendement de chimiluminescence croit jusqu'à pH 6 et qu'il décroit ensuite.

En revanche les valeurs de Tmax et de T1/2 décroissent lorsque le pH augmente au-delà de 5. L'intensité maximale augmente de façon importante avec le pH.

**TABLEAU 1**

| Réactif | Limite détection (f mol) Fluorescence | Limite détection (f mol) Chimiluminescence | Inconvénients |
|---|---|---|---|
| Luminarine-1 | 380 | TCPO :2<br>DNPO : 1 | |
| Chlorure de dansyle | 100 | TCPO : 10<br>DNPO : 5 | Toxicité, phase inversée uniquement |
| Orthophtalaldéhyde | 2100 | TCPO : 94 | Sensibilité faible |
| Fluorescamine | 500 | TCPO : 25 | Applications restreintes |
| Amino butyléthyl isoluminol | --- | 20 | Durée de réaction : 4H |
| Chlorure de nitro benzoxadiazole | 170 | TCPO : 19 | Sensibilité faible avec matériel usuel |

TABLEAU 2

| | $RF^1$ | ε 365 nm | S $10^3$ |
|---|---|---|---|
| COUMARINE-102 | 0,57 | 23 100 | 36 |
| COUMARINE-1 | 0,55 | 38 000 | 57 |
| COUMARINE-311 | 0,52 | 26 900 | 40 |

RF = rendement quantique de fluorescence.
ε = coefficient d'extinction moléculaire
S = sensibilité de fluorescence

TABLEAU 3

| | DMPO | | TCPO | |
|---|---|---|---|---|
| | $R_C$ $10^2$E.mol$^{-1}$ | $R_E$ $10^2$ | $R_C$ $10^2$E.mol$^{-1}$ | $R_E$ $10^2$ |
| COUMARINE-102 | 0,26 | 0,45 | 0,37 | 0,65 |
| COUMARINE-1 | 0,18 | 0,33 | 0,37 | 0,67 |
| COUMARINE-311 | 0,13 | 0,24 | 0,17 | 0,32 |

$R_C$ = rendement quantique de chimiluminescence
$R_E$ = rendement d'excitation
DNPO = bis-(dinitro-2,4 phényl)oxalate
TCPO = bis-(trichloro-2,4,6 phényl) oxalate.

TABLEAU 4

| ($C_{102}$) E. mol$^{-1}$ | $R_C$ x $10^4$ E. mol$^{-1}$ | Imax A.U. | Tmax min. | T1/2 min. |
|---|---|---|---|---|
| 1.4 x $10^{-3}$ | 12,0 | 301 | 0,2 | 0,10 |
| 1.4 x $10^{-6}$ | 4,14 | 75 | 0,2 | 0,15 |
| 1.4 x $10^{-7}$ | 2,31 | 31 | 0,2 | 0,12 |
| 1.4 x $10^{-8}$ | 0,40 | 3,2 | 0,2 | 0,10 |
| 1.4 x $10^{-9}$ | 0,20 | 0,3 | 0,2 | 0,08 |

TABLEAU 5

| pH | $R_C$ x $10^4$ E. mol$^{-1}$ | Imax A.U. | Tmax min. | T1/2 min. |
|---|---|---|---|---|
| 4 | 1,52 | 24 | 0,8 | 5,0 |
| 5 | 1,58 | 17 | 1,0 | 6,0 |
| 6 | 2,34 | 25 | 0,8 | 5,6 |
| 7 | 2,23 | 110 | 0,4 | 1,8 |
| 8 | 1,60 | 245 | 0,2 | 0,4 |

## Revendications

**1.** Dérivé de tétrahydro-2,3,6,7,1H,5H,11H-(1)benzopyrano (6,7,8-ij)quinolizinone, caractérisé en ce qu'il répond à la formule :

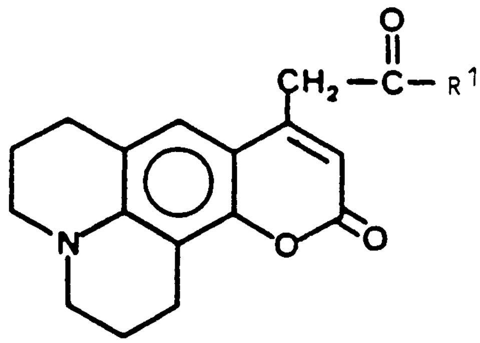

(I)

dans laquelle $R^1$ représente le radical de formule :

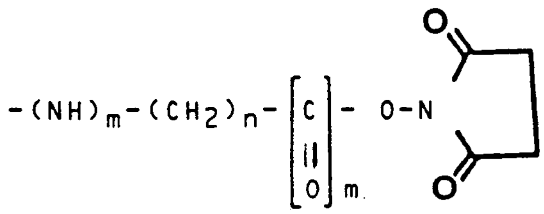

dans laquelle m est égal à 0 ou à 1, et n est égal à 0 ou est un nombre entier allant de 1 à 12 à condition que n soit égal à 0 lorsque m est égal à 0, ou le radical de formule :

$-NH-(CH_2)_n-NH_2$

dans laquelle n à la signification donnée ci-dessus.

**2.** Dérivé selon la revendication 1, caractérisé en ce que $R^1$ représente le radical :

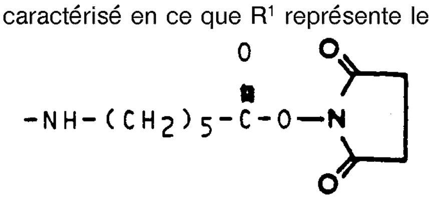

**3.** Dérivé selon la revendication 1, caractérisé en ce que $R^1$ représente le radical :

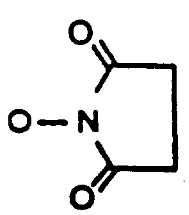

**4.** Dérivé selon la revendication 1, caractérisé en ce que $R^1$ représente le radical $-NH-NH_2$.

**5.** Dérivé selon la revendication 1, caractérisé en ce que $R^1$ représente le radical $-NH-(CH_2)_4-NH_2$

**6.** Procédé de préparation d'un dérivé de formule

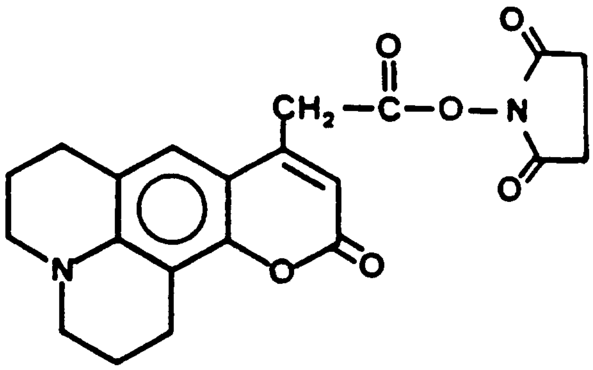

(II)

caractérisé en ce qu'il comprend les étapes successives suivantes :

1°) faire réagir la 8-hydroxyjulolidine de formule

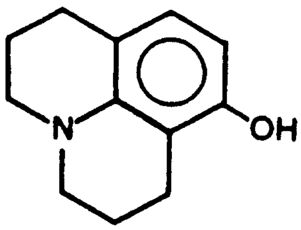

(VI)

avec un ester alkylique de l'acide oxo-3-glutarique de formule

$$R^2COO-CH_2-\underset{\underset{O}{\|}}{C}-CH_2-COOR^2$$

dans laquelle $R^2$ est un radical alkyle, pour former un composé de formule :

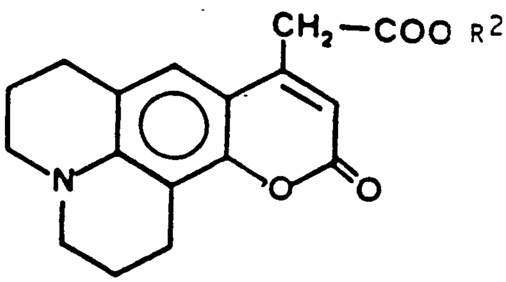

(VII)

2°) hydrolyser l'ester alkylique de formule (VII) obtenu précédemment pour obtenir l'acide de formule :

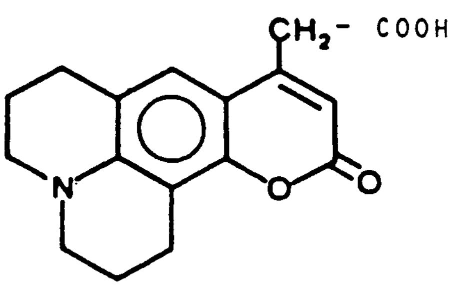

(VIII)

3°) faire réagir l'acide de formule (VIII) ainsi obtenu avec l'oxalate de dihydroxysuccinimide de formule

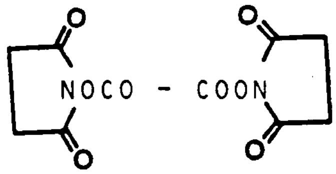

(IX)

7. Procédé selon la revendication 6, caractérisé en ce que l'ester alkylique de l'acide oxo-3-glutarique est l'ester éthylique.

8. Procédé de préparation d'un dérivé de tétrahydro-2,3,6,7,1H,5H,11H-(1)benzopyrano(6,7,8-ij)-quinolizinone-11 répondant à la formule :

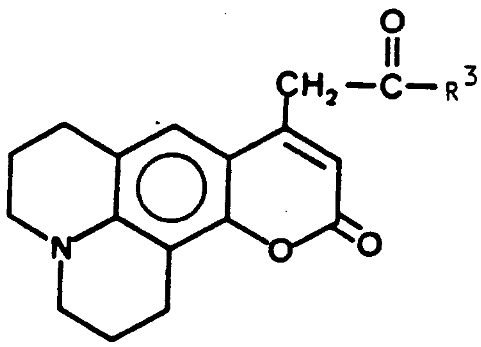

(X)

dans laquelle $R^3$ représente le radical de formule :

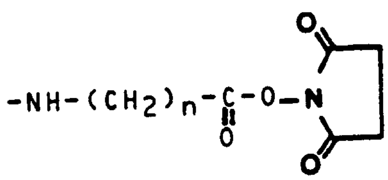

dans laquelle n est un nombre entier allant de 1 à 12,
caractérisé en ce qu'il comprend les étapes successives suivantes :
a) faire réagir le composé de formule :

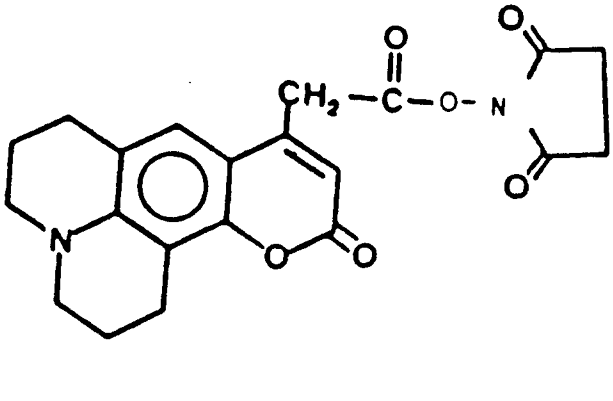

(II)

avec un aminocarboxylate d'alkyle de formule :

$_2HN-(CH_2)_n-COOR^4$

dans laquelle $R^4$ est un radical alkyle de 1 à 4 atomes de carbone et n a la signification donnée ci-dessus pour former un composé de formule :

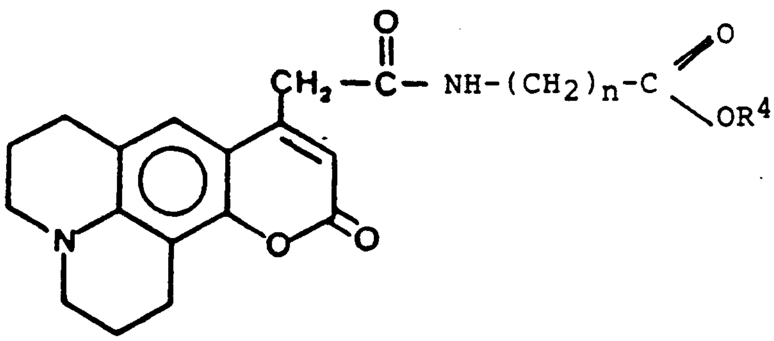

(XIII)

b) hydrolyser l'ester de formule (XIII) obtenu, pour former l'acide de formule :

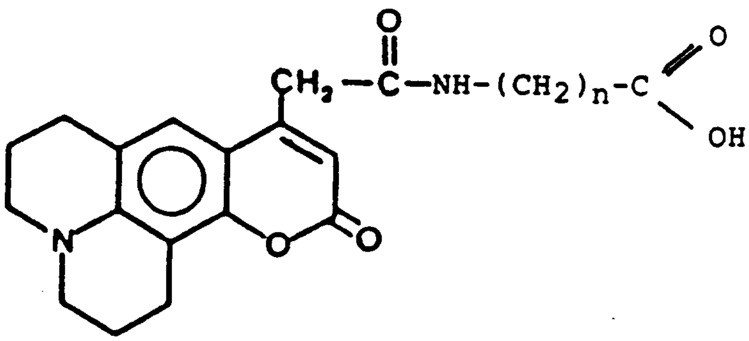

(XIV)

c) faire réagir l'acide de formule (XIV) ainsi obtenu avec

1) le carbonate de dihydroxysuccinimide de formule :

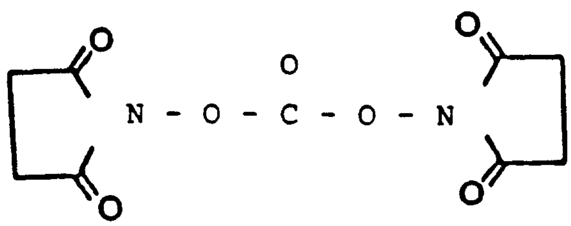

(XV)

2) l'oxalate de dihydroxy succinimide, ou

3) l'hydroxy succinimide en présence de dicyclohexylcarbodiimide.

**9.** Procédé de préparation d'un dérivé de tétrahydro-2,3,6,7,1H,5H,11H-(1)benzopyrano(6,7,8-ij)-quinolizinone-11 répondant à la formule :

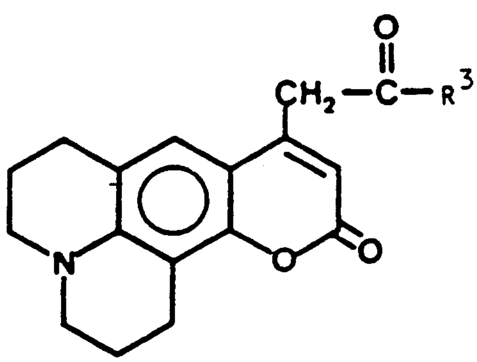

(X)

dans laquelle $R^3$ représente le radical de formule :

$-NH-(CH_2)_n-NH_2$

dans laquelle n est un nombre entier allant de 1 à 12, caractérisé en ce qu'il consiste à faire réagir le composé de formule :

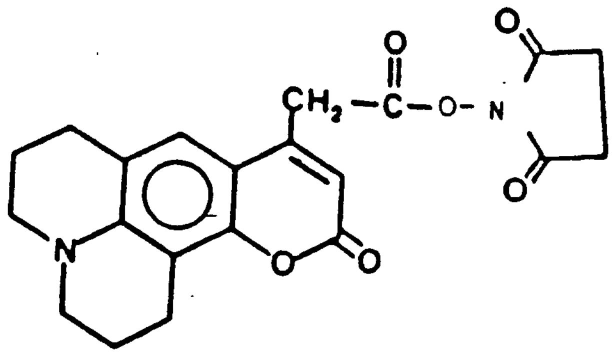

(II)

avec un diamino alcane de formule :

$_2HN-(CH_2)_n-NH_2$

dans laquelle n a la signification donnée ci-dessus.

**10.** Procédé de préparation d'un dérivé de tétrahydro-2,3,6,7,1H,5H,11H-(1) benzopyrano(6,7,8-ij)-quinolizinone-11 répondant à la formule :

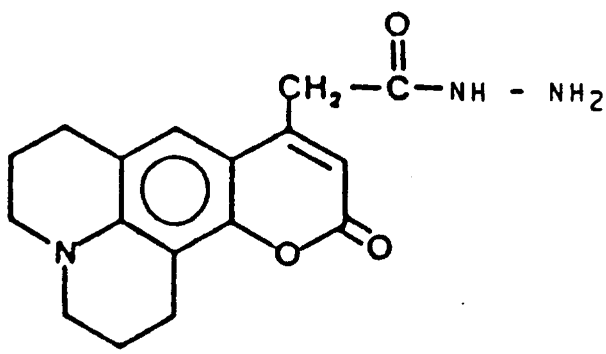

(IV)

caractérisé en ce qu'il comprend les étapes successives suivantes :

1) faire réagir la 8-hydroxyjulolidine de formule :

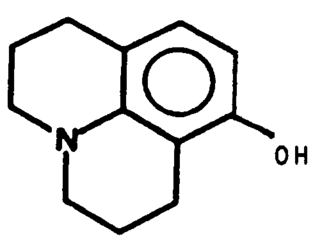

(VI)

avec un ester alkylique de l'acide oxo-3-glutarique de formule :

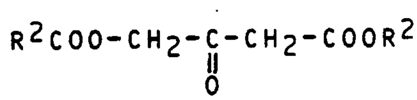

dans laquelle $R^2$ est un radical alkyle, pour former un composé de formule :

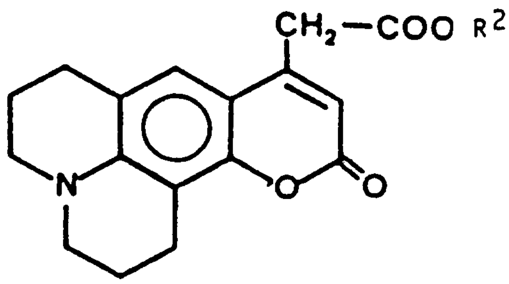

(VII)

2) faire réagir l'ester alkylique de formule (VII) ainsi obtenu avec l'hydrate d'hydrazine $_2HN-NH_2$, $H_2O$.

**11.** Procédé selon la revendication 10, caractérisé en ce que l'ester alkylique de l'acide oxo-3-glutarique est l'ester éthylique.

**12.** Procédé selon la revendication 8, caractérisé en ce que $R^3$ représente :

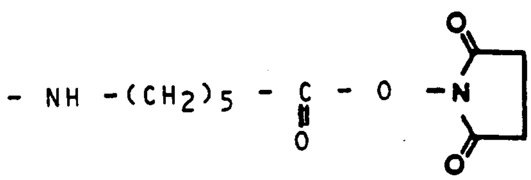

**13.** Procédé selon la revendication 9, caractérisé en ce que $R^3$ représente :

$- NH - (CH_2)_4 - NH_2$

**14.** Procédé de détection d'un composé comportant une fonction amine primaire ou secondaire par chromatographie en phase liquide, caractérisé en ce qu'il consiste à faire réagir le composé à détecter avec un dérivé répondant à la formule :

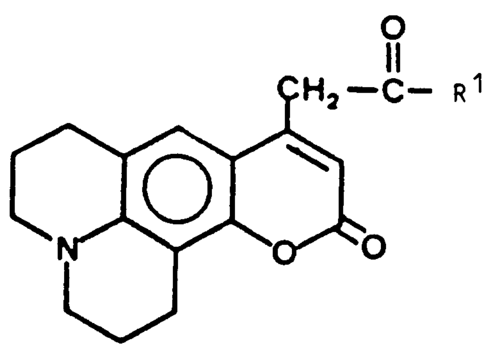

(I)

dans laquelle $R^1$ représente le radical de formule :

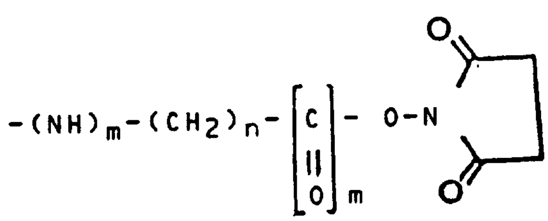

dans laquelle m = 0 ou 1 et n = 0 ou est un nombre entier allant de 1 à 12, à condition que n = 0 lorsque

m = 0, pour former un dérivé du composé à détecter, à réaliser ensuite une séparation de ce dérivé par chromatographie en phase liquide et à détecter ensuite ce dérivé par absorptiométrie, fluorimétrie, ou chimiluminescence.

**15.** Procédé selon la revendication 14, caractérisé en ce que l'on réalise la détection par chimiluminescence en utilisant un ester oxalique et du peroxyde d'hydrogène.

**16.** Procédé selon la revendication 15, caractérisé en ce que l'ester oxalique est le bis-(trichloro-2,4,6 phényl)oxalate ou le bis-(dinitro-2,4 phényl)oxalate.

## Claims

**1.** Derivative of tetrahydro-2,3,6,7,1H,5H,11H-(1)benzopyrano (6,7,8-ij)quinolizinone, characterized in that it complies with the formula:

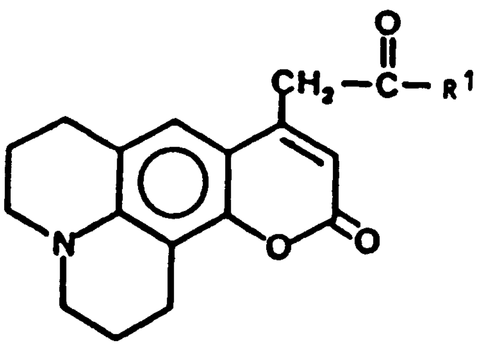

(I)

in which $R^1$ represents the radical of formula:

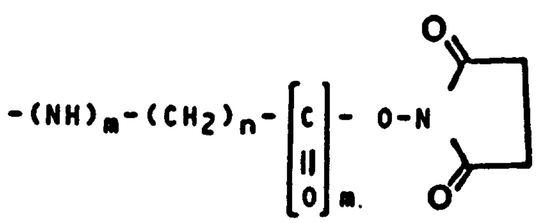

in which m is equal to 0 or to 1 and n is equal to 0 or is an integer from 1 to 12, provided that n is equal to 0 when m is equal to 0, or the radical of formula:

$-NH-(CH_2)_n-NH_2$

in which n has the meaning given hereinbefore.

**2.** Derivative according to claim 1, characterized in that $R^1$ represents the radical:

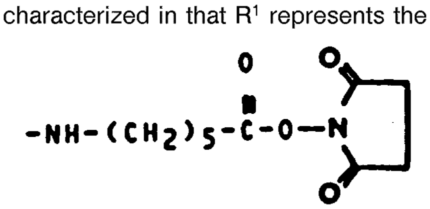

**3.** Derivative according to claim 1, characterized in that $R^1$ represents the radical:

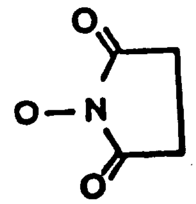

**4.** Derivative according to claim 1, characterized in that $R^1$ represents the radical $-NH-NH_2$.

**5.** Derivative according to claim 1, characterized in that $R^1$ represents the radical $-NH-(CH_2)_4-NH_2$.

**6.** Process for the preparation of a derivative of formula:

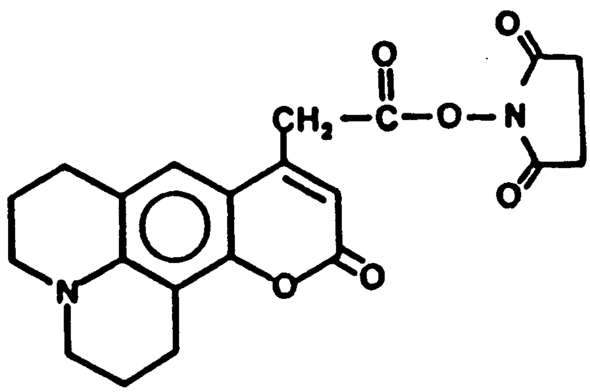

(II)

characterized in that it comprises the following successive stages:

1) reacting 8-hydroxyjulolidine of formula:

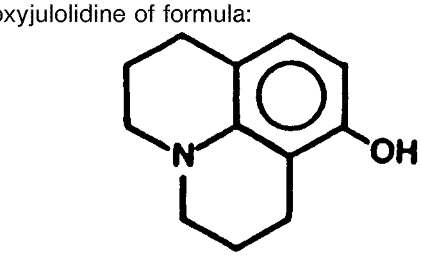

(VI)

with an oxo-3-glutaric acid alkyl ester of formula:

$R^2COO-CH_2-C-CH_2-COOR^2$
||
O

in which $R^2$ is an alkyl radical to form a compound of formula:

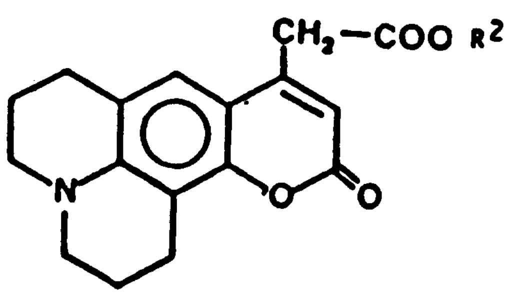

(VII)

2) hydrolyzing the previously obtained alkyl ester of formula (VII) to obtain the acid of formula:

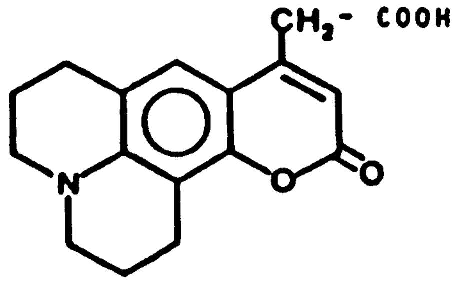

(VIII)

3) reacting the thus obtained acid of formula of (VIII) with dihydroxysuccinimide oxalate of formula:

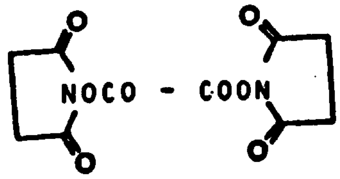

(IX)

7. Process according to claim 6, characterized in that the oxo-3-glutaric acid alkyl ester is ethyl ester.

8. Process for the preparation of a derivative of tetrahydro-2,3,6,7,1H,5H-,11H-(1)benzopyrano(6,7,8-ij)-quinolizinone-11 according to formula:

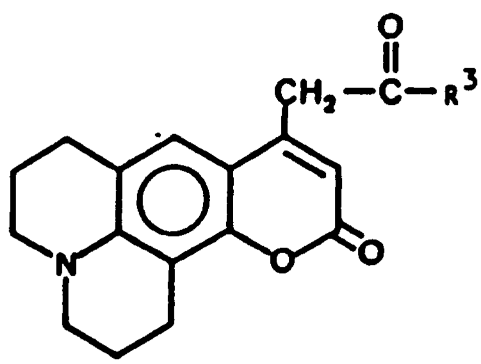

(X)

in which $R^3$ represents the radical of formula:

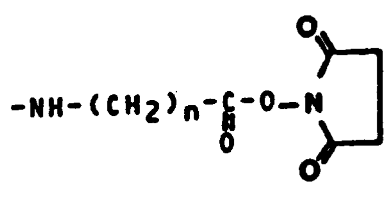

in which n is an integer from 1 to 12, characterized in that it comprises the following successive stages:

a) reacting the compound of formula:

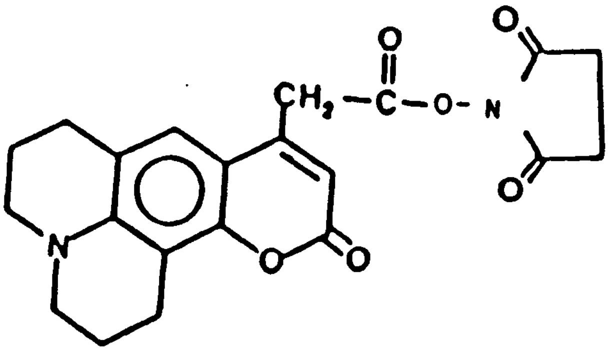

(II)

with an alkyl aminocarboxylate of formula:

$_2HN-(CH_2)_n-COOR^4$

in which $R^4$ is an alkyl radical with 1 to 4 carbon atoms and n has the meaning given hereinbefore in order to form a compound of formula:

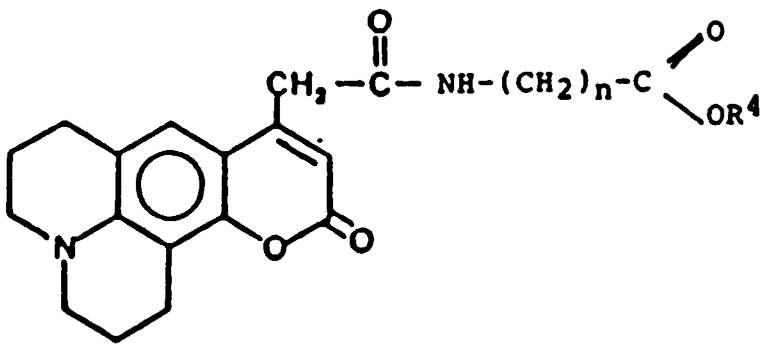

(XIII)

b) hydrolyzing the ester of formula (XIII) obtained to form the acid of formula:

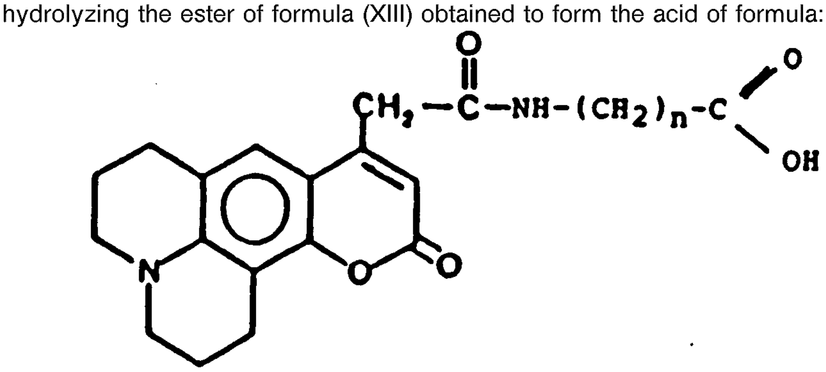

(XIV)

c) reacting the thus obtained acid of formula (XIV) with
1) dihydroxysuccinimide carbonate of formula:

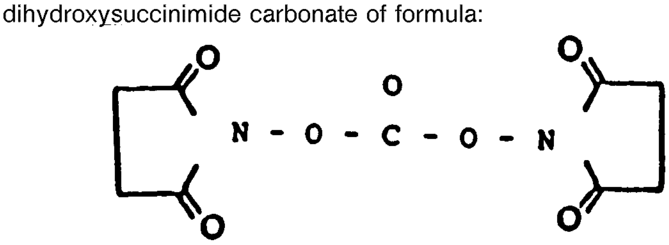

(XV)

2) dihydroxysuccinimide oxalate or
3) hydroxysuccinimide in the presence of dicyclohexyl carbodiimide.

**9.** Process for the preparation of a derivative of tetrahydro-2,3,6,7,1H,5H,11H-(1)benzopyrano(6,7,8-ij)-quinolizinone-11 according to formula:

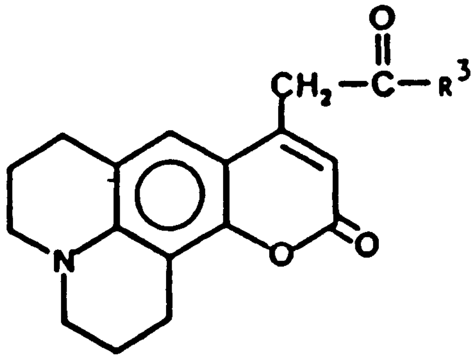

(X)

in which $R^3$ represents the radical of formula:

$-NH-(CH_2)_n-NH_2$

in which n is an integer from 1 to 12, characterized in that it comprises reacting the compound of formula:

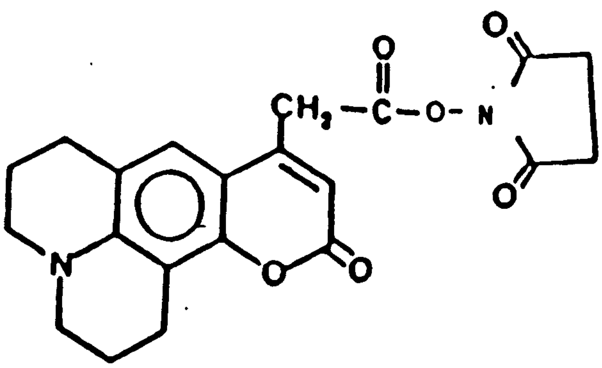

(II)

with a diaminoalkane of formula:

$_2HN-(CH_2)_n-NH_2$

in which n has the meaning given hereinbefore.

**10.** Process for the preparation of a derivative of tetrahydro-2,3,6,7,1H,5H,11H-(1)benzopyrano(6,7,8-ij)-quinolizinone-11 according to formula:

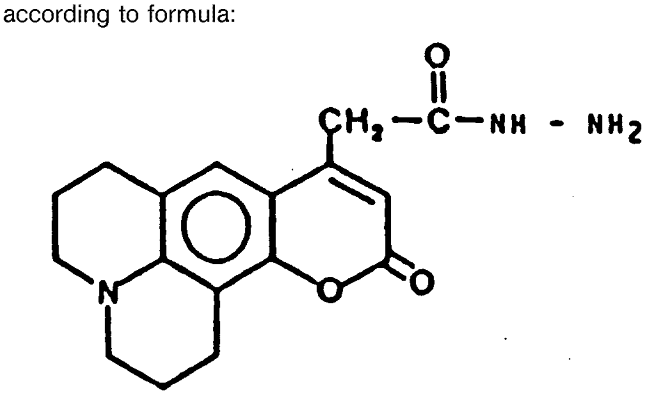

(IV)

characterized in that it comprises the following successive stages:

1) reacting the 8-hydroxyjulolidine of formula:

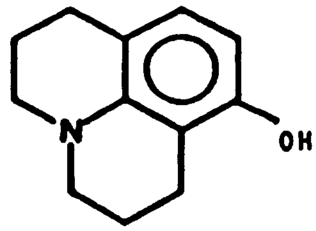

(VI)

with an oxo-3-glutaric acid alkyl ester of formula:

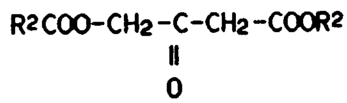

in which $R^2$ is an alkyl radical for forming a compound of formula:

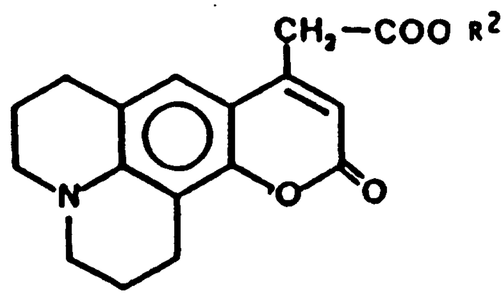

(VII)

2) reacting the alkyl ester of formula (VII) obtained in this way with hydrazine hydrate $_2HN\text{-}NH_2$, $H_2O$.

**11.** Process according to claim 10, characterized in that the oxo-3-glutaric acid alkyl ester is ethyl ester.

**12.** Process according to claim 8, characterized in that $R^3$ represents:

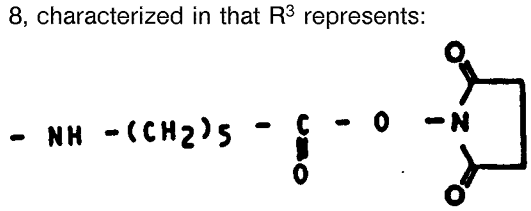

**13.** Process according to claim 9, characterized in that $R^3$ represents:

$- NH - (CH_2)_4 - NH_2$

**14.** Process for the detection of a compound having a primary or secondary amine function by liquid chromatography, characterized in that it comprises of reacting the compound to be detected with a derivative complying with the formula:

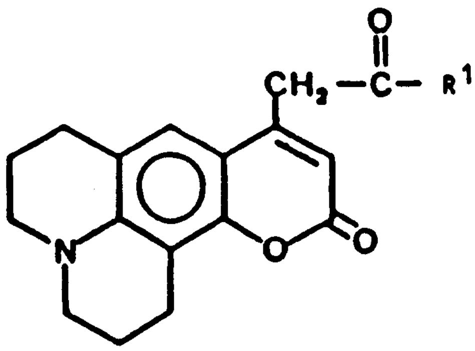

(I)

in which $R^1$ represents the radical of formula:

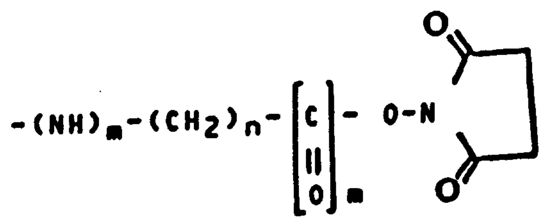

in which m = 0 or 1 and n = 0 or an integer from 1 to 12, provided that n = 0 when m = 0, to form a derivative of the compound to be detected, then carrying out a separation of said derivative by liquid chromatography and then detecting the derivative by absorptiometry, fluorimetry or chemiluminescence.

**15.** Process according to claim 14, characterized in that detection takes place by chemiluminescence using an oxalic ester or hydrogen peroxide.

**16.** Process according to claim 15, characterized in that the oxalic ester is bis-(2,4,6-trichloropbenyl)-oxalale or bis-(2,4-dinitrophenyl)-oxalate.

## Patentansprüche

**1.** Tetrahydro-2,3,6,7,1H,5H,11H-(1)Benzopyrano (6,7,8,IJ)Quinolizinon-11-Derivat,
dadurch **gekennzeichnet,**
daß es der Formel:

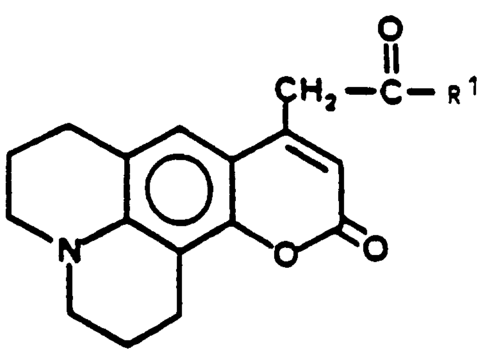

(I)

entspricht, in welcher $R^1$ das Radikal der Formel:

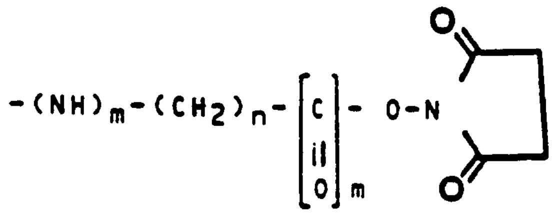

darstellt, in welchem m gleich 0 oder 1 ist, und n gleich 0 oder eine ganze Zahl ist, die von 1 bis 12 geht, unter der Bedingung, daß n gleich 0 ist, wenn m gleich 0 ist, oder das Radikal der Formel:

$-NH-(CH_2)_n-NH_2$

in welcher n die oben gegebene Bedeutung hat.

**2.** Derivat gemäß Anspruch 1, dadurch gekennzeichnet, daß $R^1$ das Radikal:

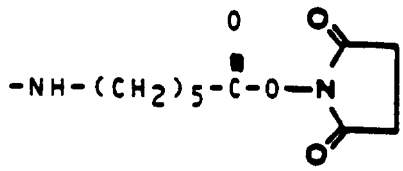

darstellt.

**3.** Derivat gemäß Anspruch 1, dadurch gekennzeichnet, daß $R^1$ das Radikal:

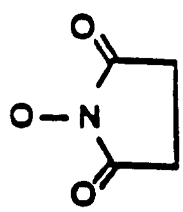

darstellt.

**4.** Derivat gemäß Anspruch 1, dadurch gekennzeichnet, daß $R^1$ das Radikal $-NH-NH_2$ darstellt.

**5.** Derivat gemäß Anspruch 1, dadurch gekennzeichnet, daß $R^1$ das Radikal $-NH-(CH_2)_4-NH_2$ darstellt.

**6.** Herstellungsverfahren eines Derivats der Formel

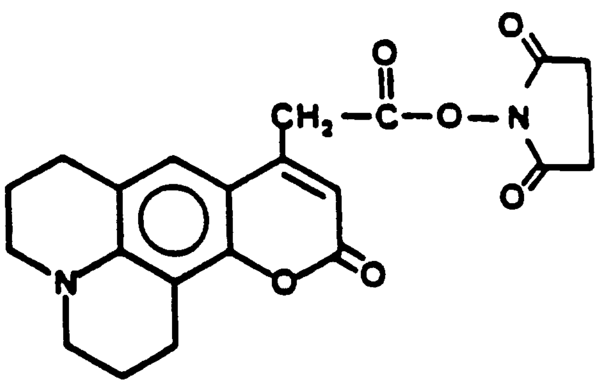

(II)

dadurch gekennzeichnet, daß es die folgenden aufeinanderfolgenden Abschnitte umfaßt:

1°) 8-Hydroxy-Julolidin der Formel

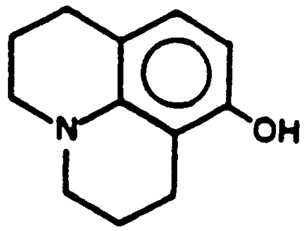

(VI)

mit einem Alkylester der Oxo-3-Glutarsäure reagieren lassen, mit der Formel

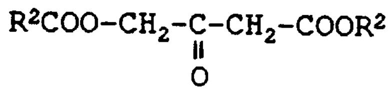

in welcher $R^2$ ein Alkylradikal ist, um eine Verbindung der Formel:

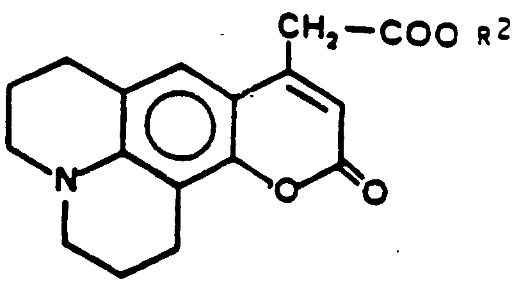

(VII)

zu formen.

2°) den vorher erhaltenen Alkylester der Formel (VII) hydrolysieren, um die Säure der Formel:

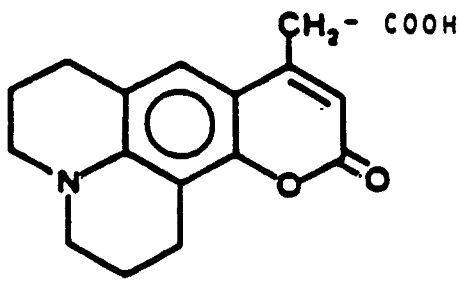

(VIII)

zu erhalten,

3°) die somit erhaltene Säure der Formel (VIII) mit Dihydroxy-Succinimid-Oxalat der Formel

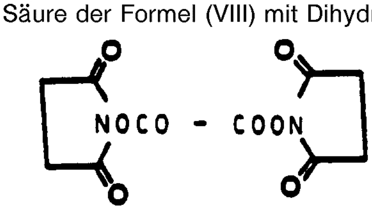

(IX)

reagieren zu lassen.

**7.** Verfahren gemäß Anspruch 6, dadurch gekennzeichnet, daß der Alkylester der Oxo-3-Glutarsäure ein Ethylester ist.

**8.** Herstellungsverfahren eines Tetrahydro-2,3,6,7,1H,5H,11H-(1)Benzopyrano(6,7,8,IJ)Quinolizinon-11-Derivats, das der Formel:

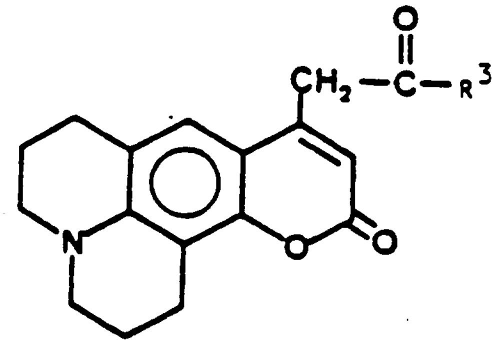

(X)

entspricht, in welcher $R^3$ das Radikal der Formel:

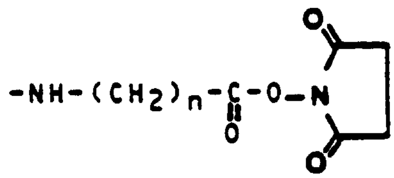

darstellt, in welcher n eine ganze Zahl zwischen 1 und 12 ist,
dadurch gekennzeichnet, daß es die folgenden aufeinanderfolgenden Abschnitte umfaßt:
a) die Verbindung der Formel:

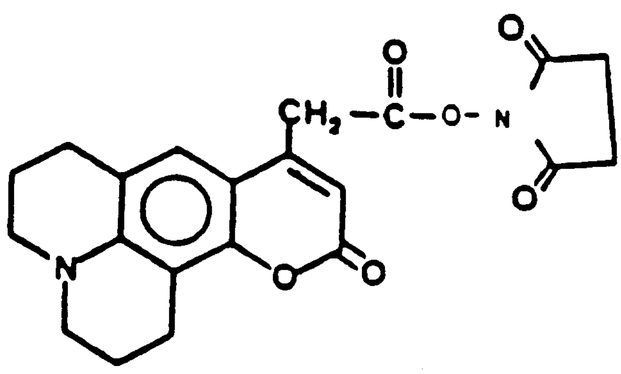

(II)

mit einem Alkyl-Aminocarboxylat der Formel:

$_2HN-(CH_2)_n-COOR^4$

reagieren lassen, in welcher $R^4$ ein Alkylradikal mit 1 bis 4 Kohlenstoffatomen ist und n die oben gegebene Bedeutung hat, um eine Verbindung der Formel:

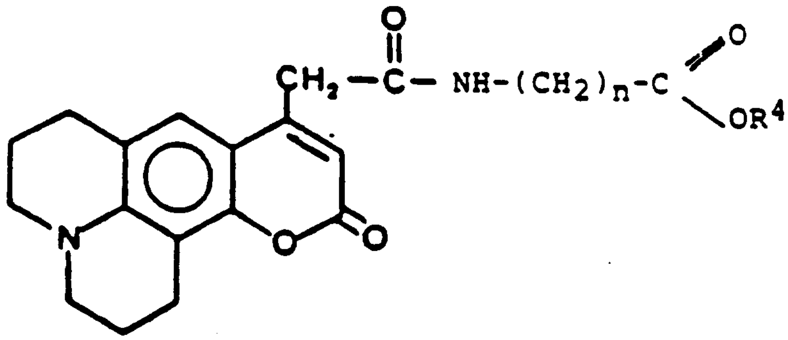

(XIII)

zu bilden,

b) den erhaltenen Ester der Formel (XIII) hydrolysieren, um die Säure der Formel:

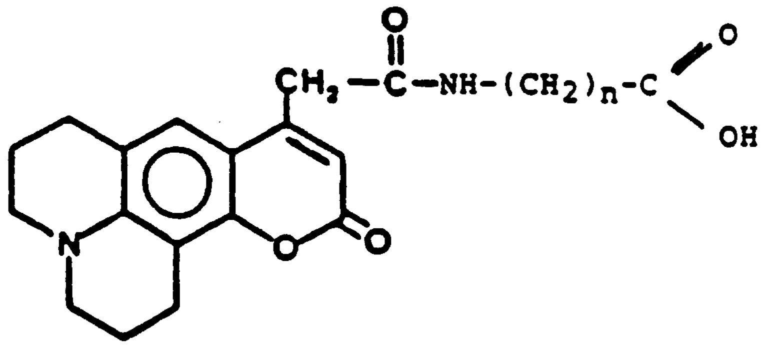

(XIV)

zu bilden,

c) die somit erhaltene Säure der Formel (XIV) mit

1) Dihydroxy-Succinimid-Carbonat der Formel:

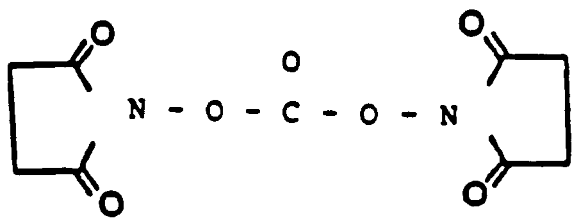

(XV)

2) Dihydroxy-Succinimid-Oxylat, oder

3) Hydroxy-Succinimid in Anwesenheit von Bicyclohexyl-Carbodiimid reagieren lassen.

**9.** Herstellungsverfahren eines Tetrahydro-2,3,6,7,1H,5H,11H-(1)Benzopyrano(6,7,8,IJ)Quinolizinon-11-Derivats, entsprechend der Formel:

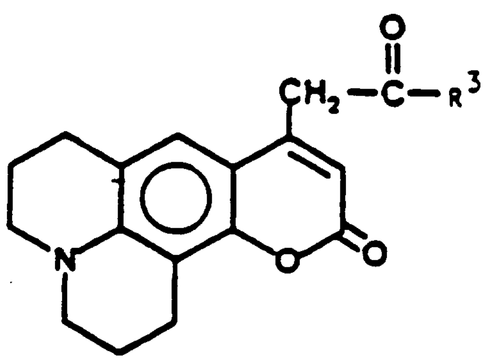

(X)

in welcher $R^3$ das Radikal der Formel:

$-NH-(CH_2)_n-NH_2$

darstellt, in welcher n eine ganze Zahl zwischen 1 und 12 ist, dadurch gekennzeichnet, daß es daraus besteht, die Verbindung der Formel:

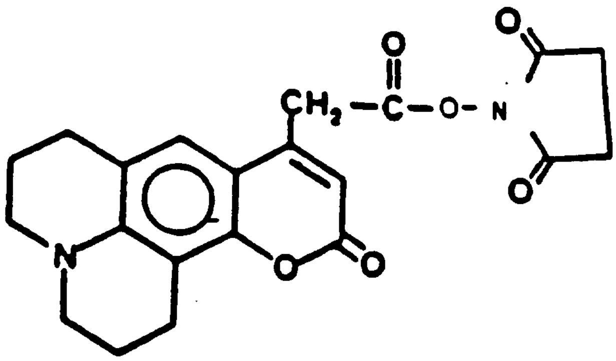

(II)

mit einem Diamino-Alkan der Formel:

$_2HN-(CH_2)_n-NH_2$

reagieren zu lassen, in welcher n die oben gegebene Bedeutung hat.

**10.** Herstellungsverfahren eines Tetrahydro-2,3,6,7,1H,5H,11H-(1)Benzopyrano(6,7,8,IJ)Quinolizinon-11-Derivats, entsprechend der Formel:

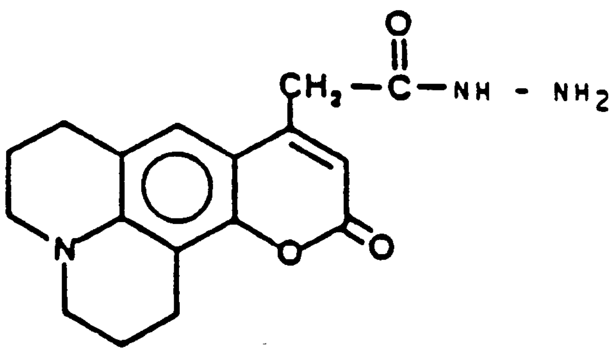

(IV)

dadurch gekennzeichnet, daß es die folgenden aufeinanderfolgenden Abschnitte umfaßt:

1) 8-Hydroxy-Julolidin der Formel:

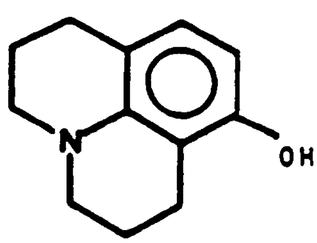

(VI)

mit einem Alkylester der Oxo-3-Glutarsäure der Formel:

$$R^2COO-CH_2-\underset{\underset{O}{\parallel}}{C}-CH_2-COOR^2$$

reagieren zu lassen, in welcher $R^2$ ein Alkylradikal ist, um eine Verbindung der Formel:

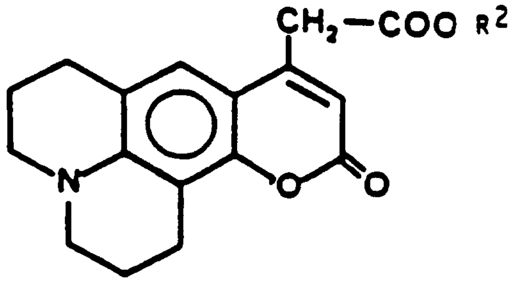

(VII)

zu bilden,

2) den somit erhaltenen Alkylester der Formel (VII) mit $_2HN\text{-}NH_2$, $H_2O$ Hydrazin-Hydrat reagieren lassen.

**11.** Verfahren gemäß Anspruch 10, dadurch gekennzeichnet, daß der Alkylester der Oxo-3-Glutarsäure ein Ethylester ist.

**12.** Verfahren gemäß Anspruch 8, dadurch gekennzeichnet, daß $R^3$:

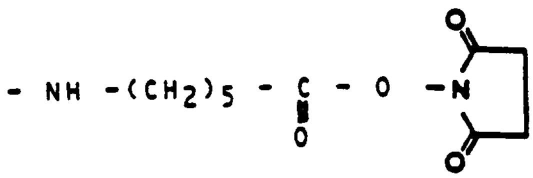

darstellt.

**13.** Verfahren gemäß Anspruch 9, dadurch gekennzeichnet, daß $R^3$:

$-NH-(CH_2)_4-NH_2$

darstellt.

**14.** Detektionsverfahren einer Verbindung, die eine primäre oder sekundäre Aminogruppe umfaßt, durch Flüssigchromatographie, dadurch gekennzeichnet, daß es daraus besteht, die zu detektierende Verbindung mit einem Derivat reagieren zu lassen, welches der Formel:

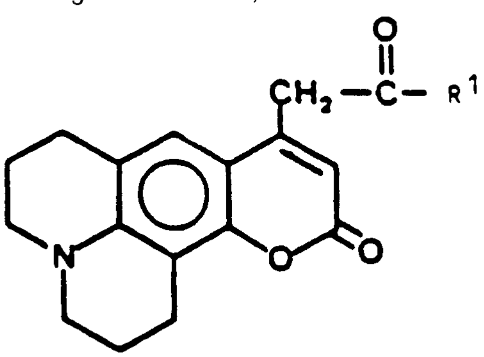

(I)

entspricht, in welcher $R^1$ ein Radikal der Formel:

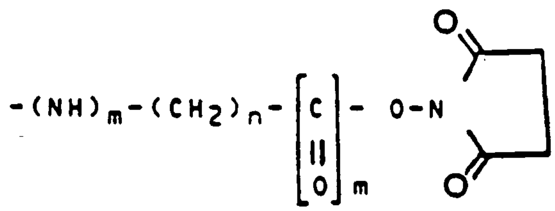

darstellt, in welcher m = 0 oder 1 und n = 0 oder eine ganze Zahl zwischen 1 und 12 ist, unter der Bedingung, daß n = 0, wenn m = 0, um ein Derivat der zu detektierenden Verbindung zu bilden und dann eine Abtrennung dieses Derivats durch Flüssigchromatographie durchzuführen, und um anschließend dieses Derivat durch Absorptionsmessung, Fluorimetrie oder Chemolumineszenz zu detektieren.

**15.** Verfahren gemäß Anspruch 14, dadurch gekennzeichnet, daß man die Detektion mittels Chemolumineszenz ausführt, indem man einen Oxalester und Wasserstoffperoxid verwendet.

**16.** Verfahren gemäß Anspruch 15, dadurch gekennzeichnet, daß der Oxalester Bis-(Trichloro-2,4,6 Phenyl)-Oxalat oder Bis-(Binitro-2,4 Phenyl)-Oxalat ist.

FIG. 1

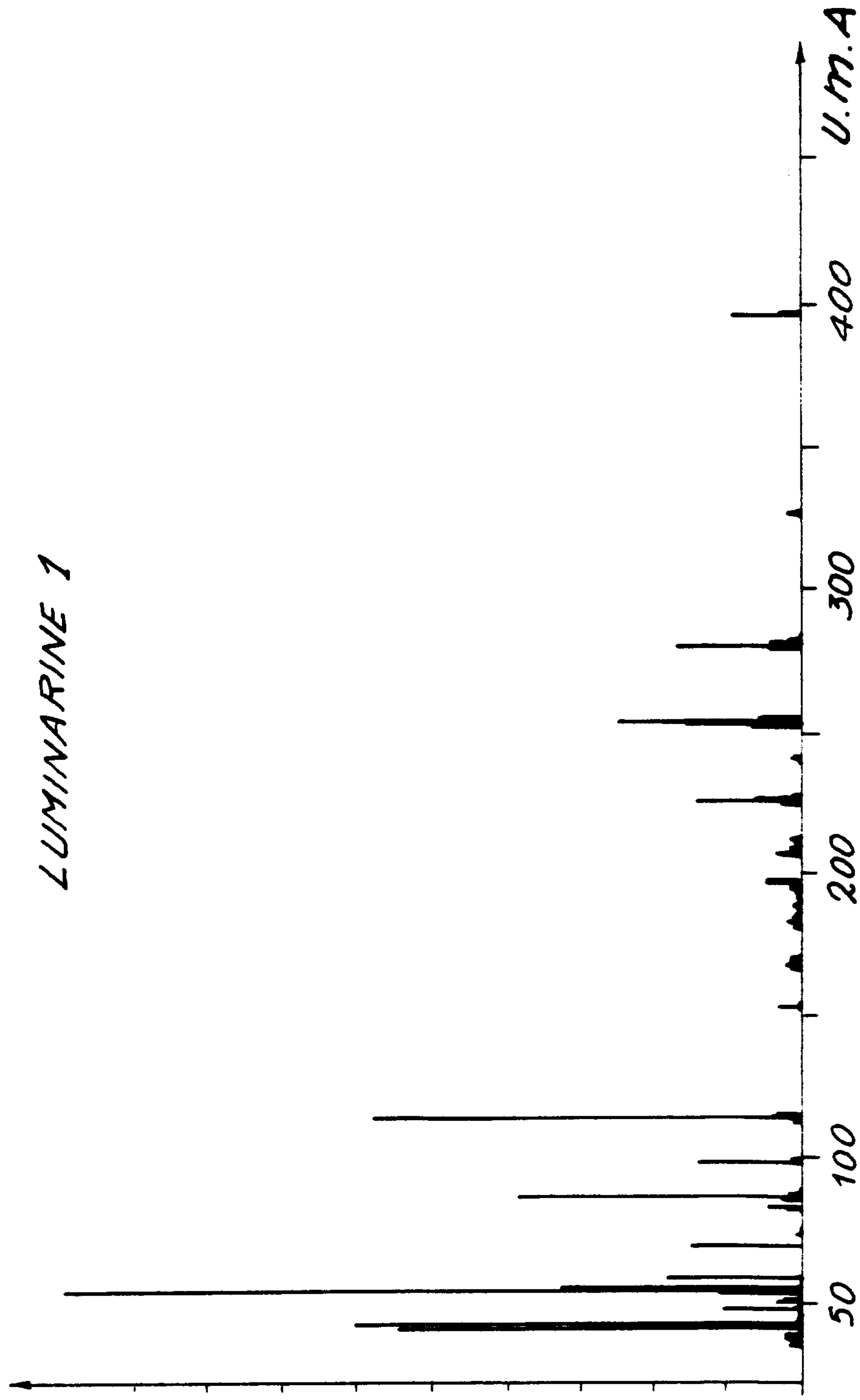

FIG. 2

LUMINARINE 1

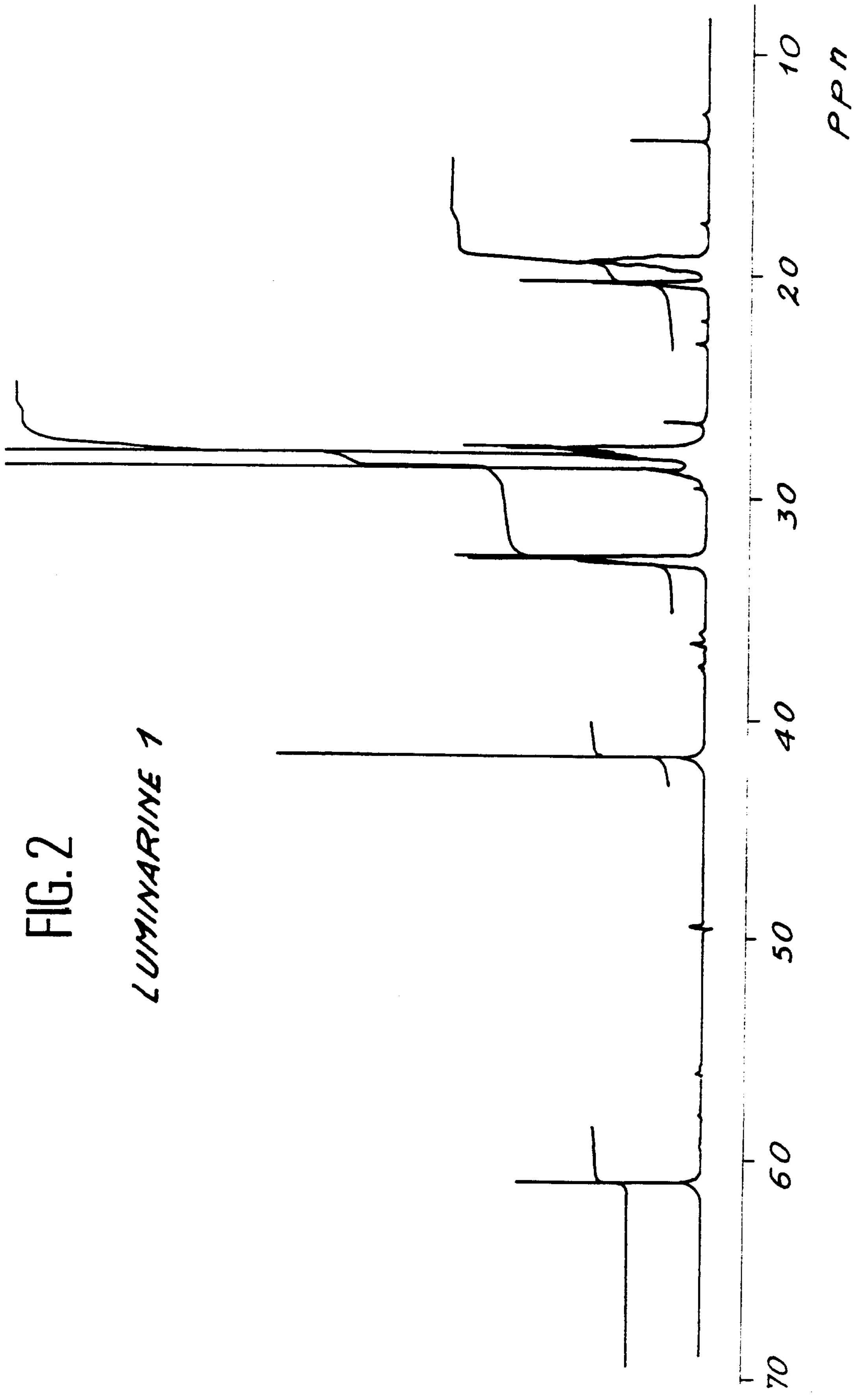

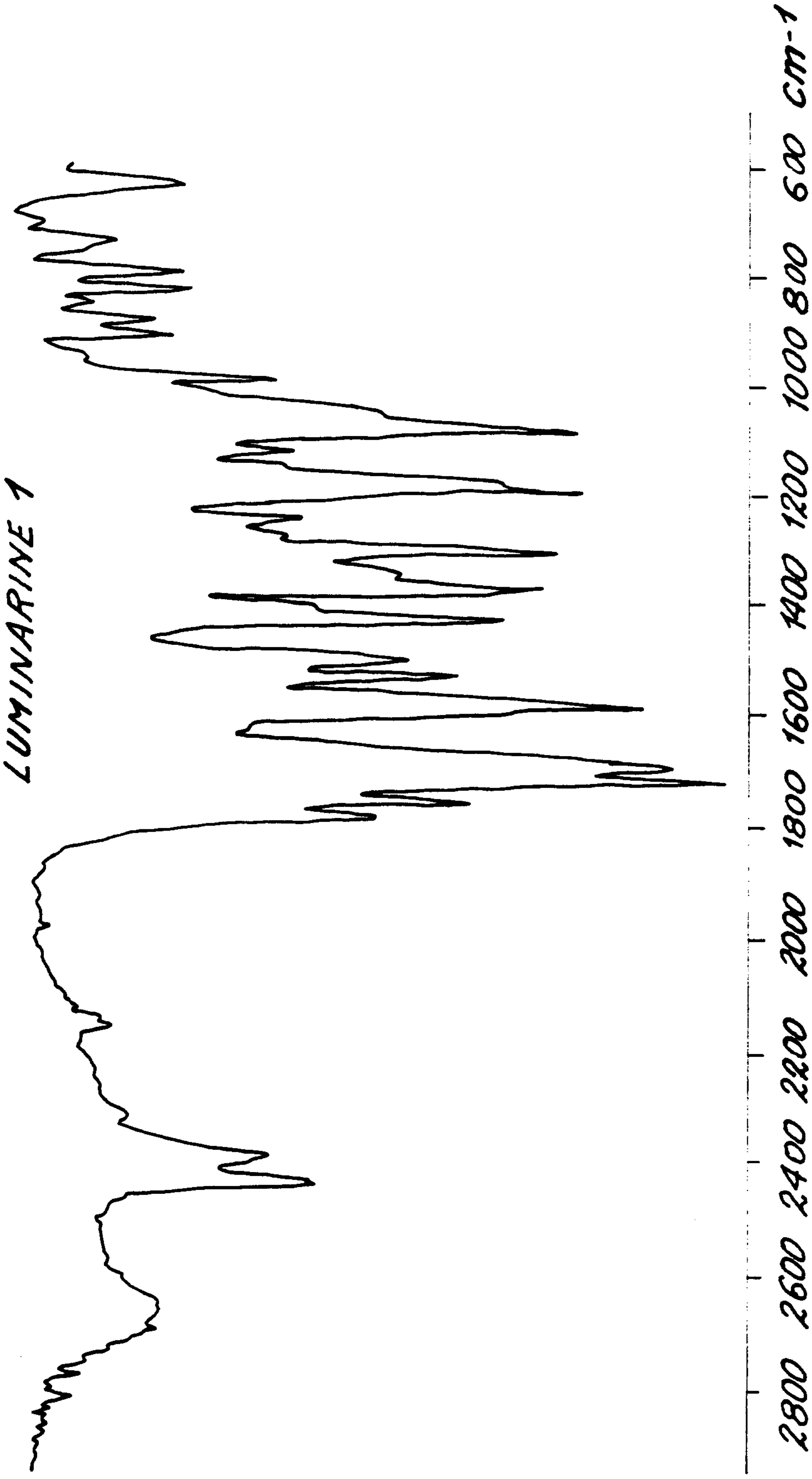
LUMINARINE 1
cm-1
600
800
1000
1200
1400
1600
1800
2000
2200
2400
2600
2800

FIG. 3

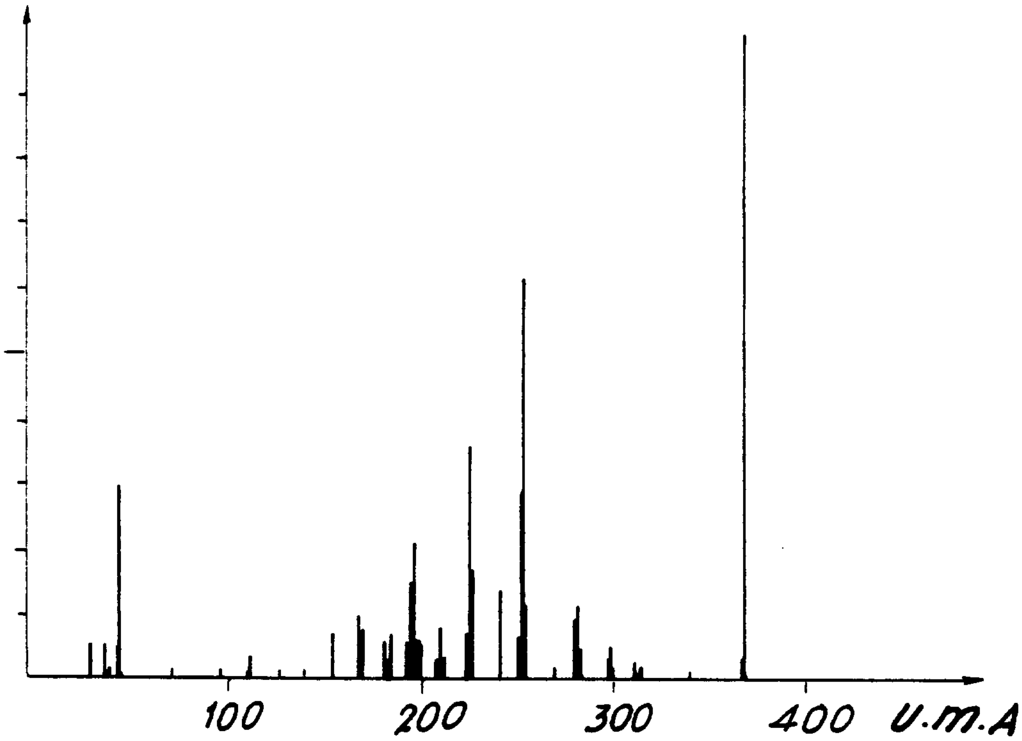


FIG. 4

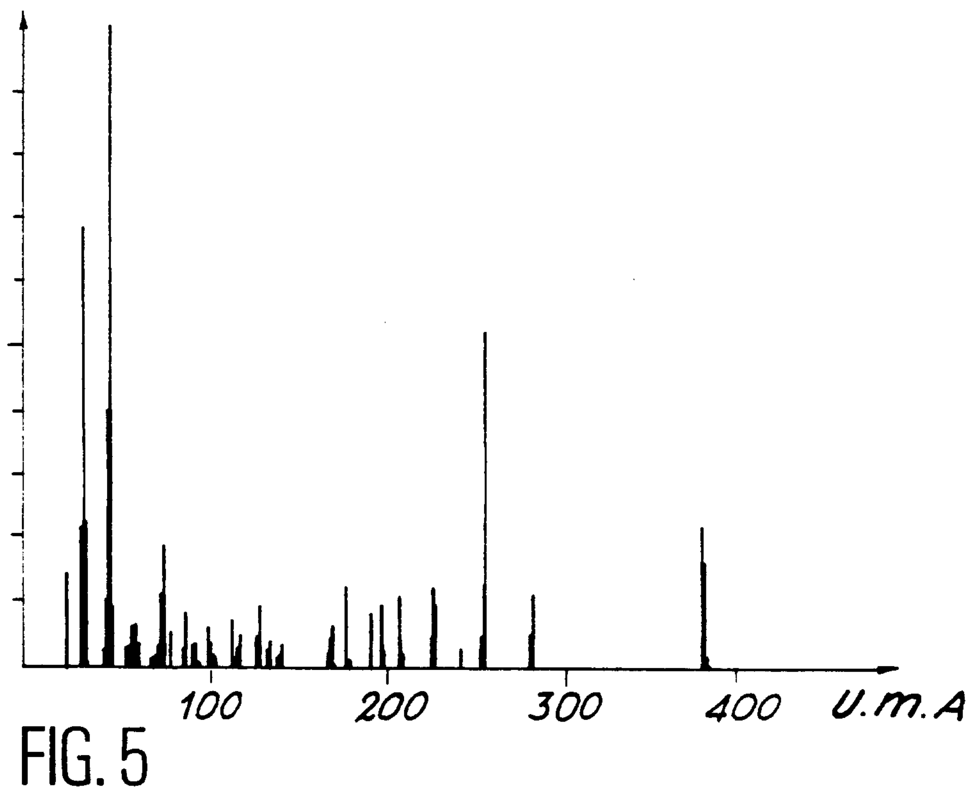


FIG. 5

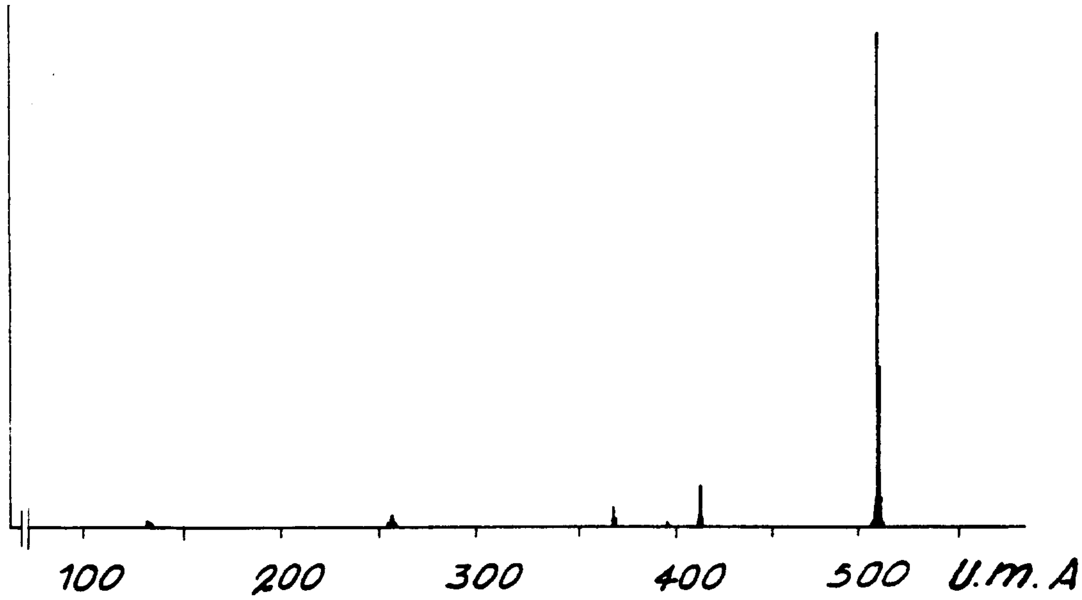


FIG. 6 LUMINARINE 2

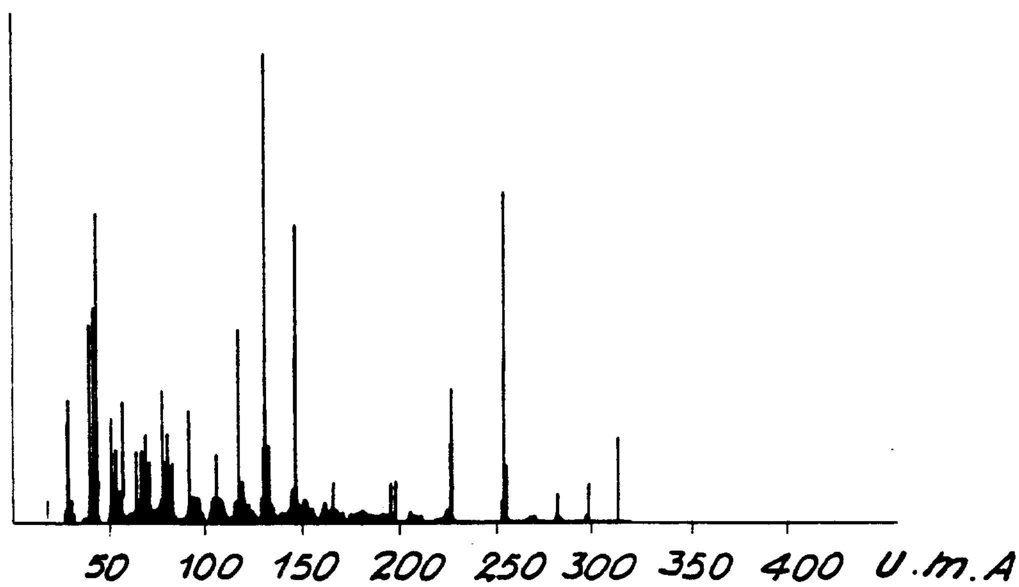


FIG. 7 LUMINARINE 3

FIG. 8

LUMINARINE 3

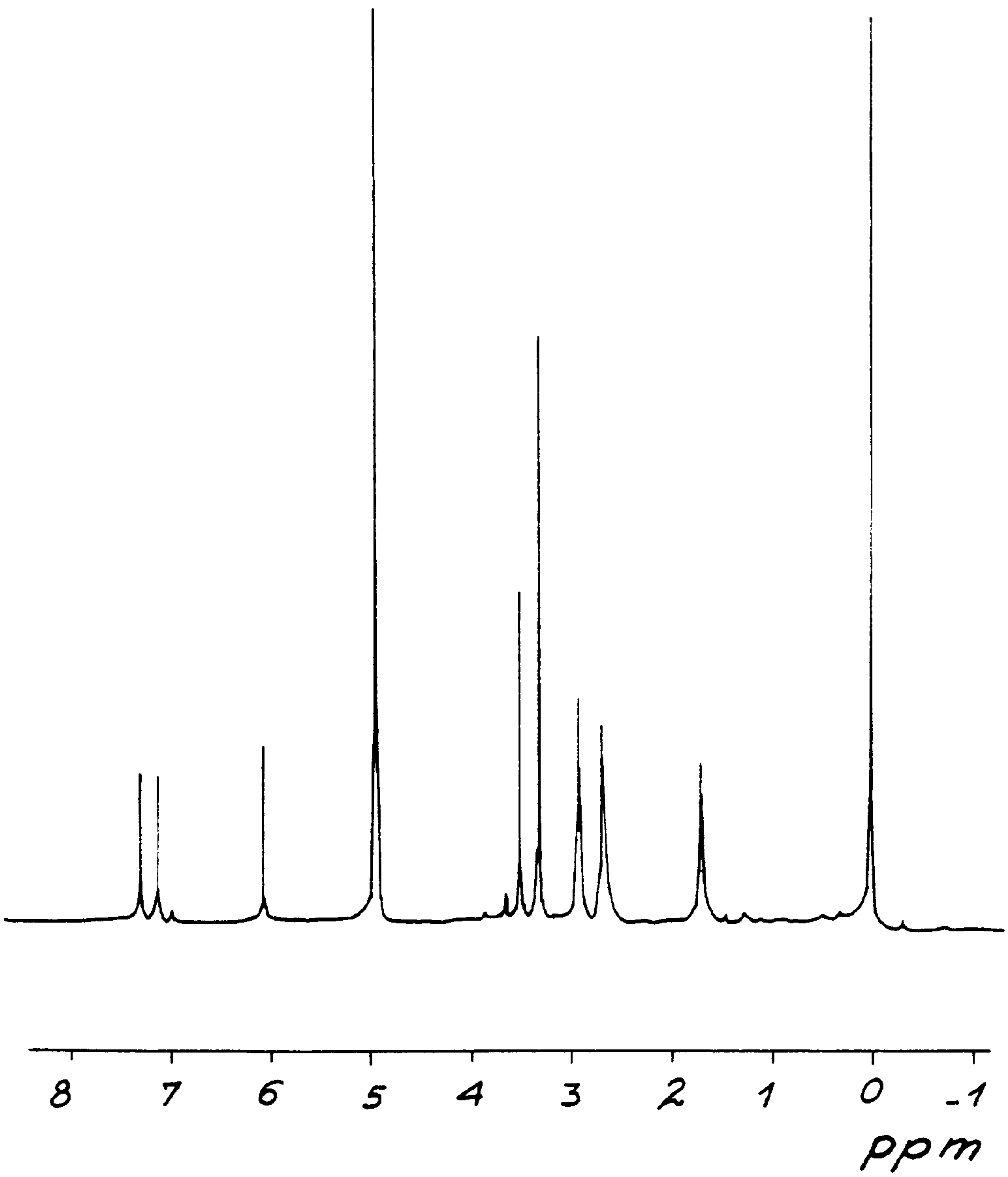

FIG 9

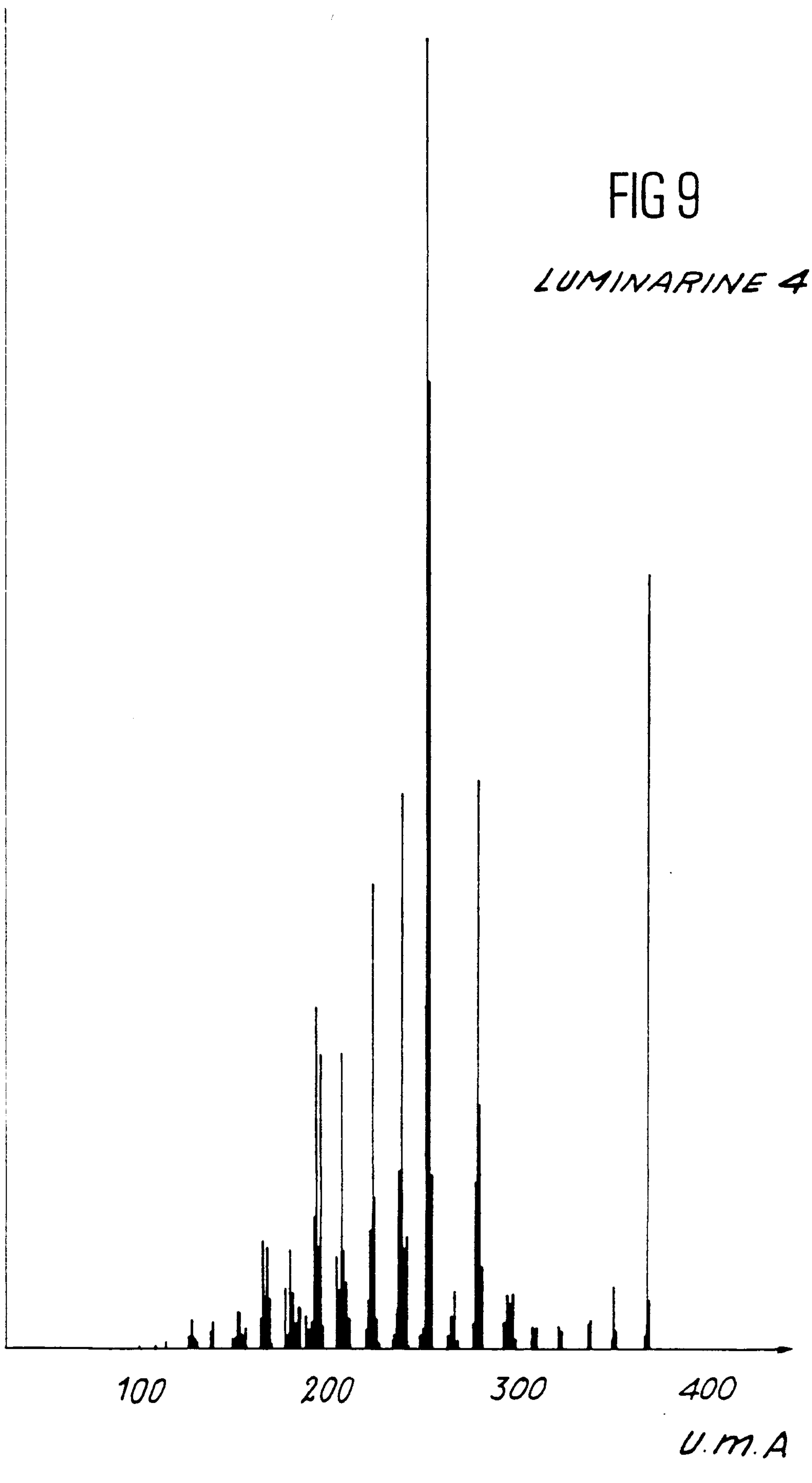

FIG. 10

LUMINARINE 4